(19)

**Europäisches Patentamt**

**European Patent Office**

**Office européen des brevets**

(11) **EP 1 586 583 A2**

(12) **EUROPEAN PATENT APPLICATION**

(43) Date of publication:
**19.10.2005 Bulletin 2005/42**

(51) Int Cl.[7]: **C07K 14/31**, A61K 38/16,
G01N 33/566

(21) Application number: **04076120.7**

(22) Date of filing: **16.04.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(71) Applicant: **Alligator Bioscience AB (publ)
223 70 Lund (SE)**

(72) Inventors:
 • **Van Strijp, Johannes, Antonius, Geradus
  3984 NV Odijk (NL)**
 • **De Haas, Carla
  3981 GH Bunnik (NL)**
 • **Kemmink, Johan
  3508 TB Utrecht (NL)**

 • **Van Kessel, Kok
  3981 EX Bunnik (NL)**

(74) Representative: **Thomas, Philip John Duval et al
Eric Potter Clarkson,
Park View House,
58 The Ropewalk
Nottingham NG1 5DD (GB)**

Remarks:
The sequence listing, which is published as annex
to the application documents, was filed after the date
of filing. The applicant has declared that it does not
include matter which goes beyond the content of the
application as filed.

(54) **Compounds that block C5a complement receptor and their use in therapy**

(57) The present invention relates to compounds that are able to prevent intramolecular contact of the N-terminal residues 10 to 18 of human C5aR with the extracellular loops thereof. More specifically the invention relates to compounds that are able to bind the aspartates in positions 10, 15 and 18 and the glycine in position 12 of the human C5aR. Such compounds are preferably of the general formula $X_n$-E-$X_{39}$-K-$X_7$-Y-V-$X_{11}$-Y-$X_m$, wherein $X_n$, $X_{39}$, $X_7$, $X_{11}$ and $X_m$ are stretches of amino acids and the other letters represent the corresponding amino acids or non-proteinogenic analogs thereof. The invention further relates to their use in prophylaxis and therapy.

**EP 1 586 583 A2**

**Description**

The present invention relates to compounds that bind the C5a receptor (C5aR) and block the inflammatory and immuno-modulatory activity mediated by endogenous C5a for treatment of inflammatory diseases and disease states. The invention further relates to the use of a C5a receptor (C5aR) N-terminal amino acid sequence for selection and development of anti-C5aR compounds.

Our innate immune system protects the body against foreign invaders (e.g. bacteria, viruses, fungi, and cancer cells). The most important cells of this system are monocytes and neutrophils. After recognition they ingest and kill invaders such as bacteria, fungi and viruses. Since they are part of the body's first line of defense, their most important task is to kill and remove the invading agent as quickly as possible. This is accomplished through very aggressive substances (e.g. free radicals and enzymes) that are not only lethal to the invader, but also cause damage to host cells in the vicinity. Substances from these damaged cells and the locally activated cells from the innate system itself will further attract increasing numbers of neutrophils and monocytes, causing local inflammation. The inflammation will subside once all the invaders have been killed and removed, together with the various cells killed in the process. Healing of the wound, caused by the inflammatory response, can then begin.

In some cases, such an aggressive and damaging inflammatory reaction, caused by over-activated neutrophils, is unnecessarily strong or even starts without the presence of an infectious trigger. In some cases this inflammatory response is responsible for serious, sometimes lethal disorders and includes conditions like Adult Respiratory Distress Syndrome (ARDS), severe tissue damage following thrombotic events such as heart attacks and stroke, inflammatory bowel diseases and rheumatoid arthritis.

The complement (C) system is a key component of the innate immune system, playing a central role in host defense against pathogens. It is also a powerful drive to initiate inflammation and can, if unregulated, cause pathology leading to severe tissue damage. When bacteria have invaded the body and, for example, infected the central nervous system (as in meningitis) substances of microbial origin lead to the activation of a complement factor 5 (C5) convertase enzyme-complex, that converts C5 of the complement system into its activated C5a form. C5a is a chemo-attractant: a substance that can activate and attract cells from the blood vessels (the migration process). Neutrophils are responsive to these two substances and also to interleukin-8 (IL-8).

Activated neutrophils can easily migrate from blood vessels. This is because the chemo-attractants, microbial products and substances from activated monocytes will have increased the permeability of the vessels and stimulated the endothelial cells of the vessel walls to express certain adhesion molecules. Neutrophils express selectins and integrins (e.g. CD11b/CD18) that bind to these adhesion molecules. Once the neutrophil has adhered to the endothelial cells, it is able to migrate through the cells, under the guidance of chemo-attractants/chemokines, towards the site of infection, where the concentration of these substances is at the highest.

These substances also activate neutrophils to produce a range of other molecules, some of which attract more neutrophils (and subsequently monocytes), but, mostly, they are responsible for destroying the invading bacteria. Some of these substances (e.g. free radicals, enzymes that break down proteins (proteases) and cell membranes (lipases)) are so reactive and non-specific that cells from the surrounding tissue (and the neutrophils themselves) are destroyed, causing tissue damage. This damage is exacerbated by the presence of blood-derived fluid which has transgressed the leaky vessel wall and is responsible for the swelling that always accompanies inflammation (called edema). The pressure build up caused by this excess fluid results in further cell damage and death.

Later in the inflammatory process, monocytes migrate to the scene and become activated. Besides their role in removing bacteria and cell debris, they also produce substances such as tumor necrosis factor (TNF) and IL-8, which in turn attract more activated neutrophils, causing further local damage. TNF also has a direct stimulatory effect on neutrophils. Once all the invaders have been removed, the inflammatory response will subside and the area will be cleared of the remaining 'casualties'. Then the process of wound healing starts. In general, damaged tissue will be replaced by scar tissue formed mainly of fibroblasts and collagen. When inflammation occurs in areas of the body with an important function, like tissues formed from heart muscle cells, brain cells or lung alveolar cells, normal function will be compromised by the resulting scar formation, causing serious conditions like heart failure, paralysis and emphysema. To minimize the debilitating consequences of these conditions, it is important to 'dampen' the inflammatory reaction as quickly as possible.

Intervention to control the acute early phase inflammatory response presents an opportunity to improve the prognosis for a wide range of patients whose symptoms can be traced back to such an event. Such an approach has been advocated for many acute and chronic inflammation based diseases and shown to have potential based on findings from relevant disease models. Classical anti-inflammatory drugs such as steroids and Non Steroid Anti-Inflammatory Drugs (NSAIDS) do not have the ideal profile of action, either in terms of efficacy or safety. Steroids affect the 'wrong' cell type (monocytes) and their dampening effects are easily bypassed. NSAIDS generally show a relatively mild effect partly because they intervene at a late stage in the inflammatory process. Both classes of drugs produce a range of undesirable side effects resulting from other aspects of their pharmacological activity. Drugs acting directly and specifically to prevent migration and activation of neutrophils may have a number of advantages. Several drugs under early development only interfere with one individual aspect of neutrophil activation (e.g. C5 convertase inhibitors, antibodies against C5a, C5a-receptor blocking drugs) and migration (antibodies against integrins (like CD11b/CD18) and L-selectin on neutrophils and antibodies against adhesion molecules (like ICAM-1 and E-selectin) on endothelial cells). Antibodies against TNF and IL-8 have effects in chronic inflammation, but only marginal effects in acute inflammation, because of the minimal role monocytes (which are mainly responsible for these substances' production) play in the acute phase.

Sometimes, the cause of the acute inflammation cannot be removed and the inflammation becomes chronic. With the exception of tuberculosis, chronic hepatitis and certain other conditions, this is seldom the case with infections. However, chronic inflammation can also be caused by stimuli other than bacteria, such as auto-immune reactions. Research has shown that in chronic inflammation the role of monocytes is much more prominent, and that neutrophil migration and activation, monocyte migration and activation, tissue damage, removal of dead cells and wound healing are all going on at the same time. This complex cascade of interactions between cells and many different cytokines and chemokines has been the subject of intensive research for many years. It was believed that monocytes and their products were the most important elements that needed to be inhibited to dampen chronic inflammation. This explains why steroids, which specifically interact with monocytes, are generally more effective in chronic as opposed to acute inflammation. Long-term treatment with steroids however, is not a desirable option, because severe and unacceptable side effects can occur at the doses required to produce a clinical effect. Newer treatments using antibodies against TNF or IL-8 have shown good results, and were initially seen as proof of the major role monocytes were thought to play in chronic inflammation. Recent research casts doubts on an exclusive role for monocytes in inflammation and points to a critical role for neutrophils, which are now seen to represent better targets for therapeutic intervention.

The underlying cause of a chronic inflammatory condition is not always clear, and the original cause may not always be responsible for future recurrence. Some scientists believe that in certain chronic inflammatory diseases there is a continuous cycle of events. Their idea is that existing activated neutrophils and monocytes continuously attract and activate new groups of cells, thus perpetuating the inflammatory response even when the initial stimulus is no longer present. This would suggest that an acute or periodic treatment with an effective inhibitor of the neutrophil and monocyte activation would stop the cycle of new cell recruitment, leading in due course to modification of disease progression, or even a complete cure, and not just symptomatic relief.

It is desirable to find alternative compounds that can be used in the treatment of inflammation.

One promising candidate was a new agent with inflammation-inhibiting properties that was found in the extracellular medium of growing *Staphylococcus aureus (S. aureus),* named CHemotaxis Inhibitory Protein from *Staphylococcus aureus* (CHIPS), as described in WO00/02913. CHIPS was found to inhibit fMLP- and C5a-induced activation of neutrophils, among which the fMLP- and C5a-induced chemotaxis of neutrophils. Using FITC-labeled CHIPS it was discovered that CHIPS binds both the C5aR and the FPR, using different domains. These receptors belong to the family of chemokine receptors that are all members of the superfamily of 7-transmembrane, heterotrimeric G protein coupled receptors (GPCR). Neutrophils bear several members of this receptor family on their surface including the receptors for formylated peptides, C5a, C3a, PAF, and LTB4 as well as the chemokine receptors CXCR1 and CXCR2 that recognize IL-8 and NAP/GRO/ENA, respectively. GPCR exist of an extracellular N-terminal part, three extra- and three intra-cellular loops and an intracellular C-terminal part.

It was however contemplated that the complete molecule is too large for use in therapy. In addition, it was found that when CHIPS was administered to individuals it led to the formation of immune complexes because of pre-existing antibodies against this bacterial virulence factor.

It is therefore the object of the present invention to provide alternative compounds with inflammation-inhibiting properties.

The present invention is based on the study of the interaction of CHIPS with the C5aR and thus the inhibition of C5a mediated activation. From literature it is known that C5a binds to its receptor at two distinct sites, a recognition site and an activation site. The recognition site is thought to lie in the segment of residues 21-30 of the N-terminal of the C5aR. Yet, the aspartates, present at the positions 10, 15, 16 and 18 of the C5aR N-terminal also seem critical for the binding affinity of the receptor for C5a.

These results led the present inventors to the concept that C5a binds to a recognition site, consisting of the amino acids 21-30, and that the conformation thus obtained is stabilized by the intramolecular contact of the C5aR N-terminal residues 10-18 with the extracellular loops of the C5aR. This binding and stabilization places C5a in the proper position to activate the receptor via its C-terminal activation site.

The inventors used two different expression systems, in which the whole C5aR and the C5aR N-terminus alone were expressed in HEK293 cells. With that it was discovered that CHIPS mainly binds to the C5aR N-terminus.

Site-directed mutagenesis studies of the C5aR N-terminus surprisingly proved that the CHIPS-binding site was not situated at the C5a recognition site (amino acids 21-30). CHIPS seems to prevent the intramolecular contact of the C5aR N-terminal residues 10-18 with the extracellular loops of the C5aR by binding to this region of amino acids 10 to 18. The aspartates on position 10, 15, and 18 (D10, D15, and D18) and the glycine on position 12 (G12) play an important role herein.

Site-directed mutagenesis in the N-terminus of the complete C5aR shows that D18 and G12 are the most important amino acids involved in binding of CHIPS, but that at least a combination of two mutations of the aspartates is necessary to completely inhibit binding of CHIPS.

Amino acids 38 to 350 of the C5aR are not involved in the binding of CHIPS.

Furthermore, using a sulfonated C5aR-derived peptide, comprising amino acids 7 to 28 (called herein "SO$_4$-C5AR pep7-28"), in a nuclear magnetic resonance (NMR), in which C5aR-pep7-28 was titrated to $^{15}$N-labeled CHIPS$\Delta$30 (CHIPS minus the first 30 amino acids), the exact amino acids of CHIPS$\Delta$30 involved in the binding to the C5aR-N-terminus were discovered. The most clearly involved amino acids are R46, K50, K51, G52, K54, E60, K100, K101, G102, K105, Y108, V109, and Y121, but the following amino acids might be involved in the binding of CHIPS to the C5aR-N-terminus too: T37, L38, R44, L45, N47, Y48, L49, T53, A57, F59, K61, V63, 164, L65, Y71, T73, L76, L80, D83, R84, K85, E88, L89, G91, M93, T96, Y97, E103, I110, N111, and K115.

Preliminary experiments using two CHIPS mutants, containing lysine mutations K50, K51 + K54 and K100, K101 + K105, indicate indeed that both these lysine regions are involved in the binding of CHIPS to the C5aR.

The present invention is thus based on the information about the exact binding site of CHIPS to the C5aR. Moreover, it provides information about the amino acids within the CHIPS molecule that are involved in the binding to the C5aR. This dual discovery is then used for the development of anti-C5aR compounds, that may be CHIPS-derived compounds or non-CHIPS-derived compounds.

The invention thus relates to compounds that are able to prevent intramolecular contact of the N-terminal residues 10 to 18 of human C5aR with the extracellular loops thereof. In particular, the invention relates to compounds that bind to the following residues in the human C5a receptor (C5aR): aspartates in positions 10, 15, and 18 (D10, D15, and D18) and the glycine on position 12 (G12).

The present invention more specifically relates to compounds of the general formula:

$$X_n\text{-}E\text{-}X_{39}\text{-}K\text{-}X_7\text{-}Y\text{-}V\text{-}X_{11}\text{-}Y\text{-}X_m \qquad\qquad (I)$$

wherein:

$X_n$ may or may not be present and is a stretch of proteinogenic amino acids, non-proteinogenic amino acids, D-amino acids, peptidomimetic building blocks or combinations thereof;
K is lysine or a non-proteinogenic analog thereof;
E is glutamic acid or a non-proteinogenic analog thereof;
Y is tyrosine or a non-proteinogenic analog thereof; and
V is valine or a non-proteinogenic analog thereof
$X_{39}$, $X_7$, and $X_{11}$ are each a stretch of 39, 7 or 11, respectively, proteinogenic amino acids, non-proteinogenic amino acids, D-amino acids, peptidomimetic building blocks or combinations thereof;
$X_m$ may or may not be present and is a stretch of proteinogenic amino acids, non-proteinogenic amino acids, D-amino acids, peptidomimetic building blocks or combinations thereof;
and have the same three-dimensional conformation as naturally occurring CHIPS.

More specifically, the invention relates to compounds of the general formula:

$$X_n\text{-}R\text{-}X_3\text{-}K\text{-}K\text{-}G\text{-}X\text{-}K\text{-}X_5\text{-}E\text{-}X_{39}\text{-}K\text{-}K\text{-}G\text{-}X_2\text{-}K\text{-}X_2\text{-}Y\text{-}V\text{-}X_{11}\text{-}Y\text{-}X_m \qquad\qquad (II)$$

wherein:

$X_n$ may or may not be present and is a stretch of proteinogenic amino acids, non-proteinogenic amino acids, D-amino acids, peptidomimetic building blocks or combinations thereof;
R is arginine or a non-proteinogenic analog thereof;
K is lysine or a non-proteinogenic analog thereof;
E is glutamic acid or a non-proteinogenic analog thereof;
X, $X_2$, $X_{31}$ $X_5$, $X_{11}$ and $X_{39}$ are each a stretch of 1, 2, 3, 5, 11 and 39, respectively, proteinogenic amino acids, non-proteinogenic amino acids, D-amino acids, peptidomimetic building blocks or combinations thereof;
Y is tyrosine or a non-proteinogenic analog thereof; and

V is valine or a non-proteinogenic analog thereof

$X_m$ may or may not be present and is a stretch of proteinogenic amino acids, non-proteinogenic amino acids, D-amino acids, peptidomimetic building blocks or combinations thereof;

and having the same three-dimensional conformation as naturally occurring CHIPS.

In a particular embodiment of the invention the compounds are of the general formula:

$$X_n\text{-}T\text{-}X_6\text{-}R\text{-}L\text{-}R\text{-}N\text{-}X_2\text{-}K\text{-}K\text{-}G\text{-}T\text{-}K\text{-}X_4\text{-}F\text{-}E\text{-}K\text{-}X\text{-}V\text{-}X_7\text{-}Y\text{-}X_4\text{-}L\text{-}X_{11}\text{-}E\text{-}X_{11}\text{-}K\text{-}K\text{-}$$

$$G\text{-}E\text{-}X\text{-}K\text{-}X_2\text{-}Y\text{-}V\text{-}X\text{-}N\text{-}X_3\text{-}K\text{-}X_5\text{-}Y\text{-}X_m \qquad\qquad (III)$$

wherein

$X_n$ may or may not be present and is a stretch of proteinogenic amino acids, non-proteinogenic amino acids, D-amino acids, peptidomimetic building blocks or combinations thereof;

T is threonine or a non-proteinogenic analog thereof;

R is arginine or a non-proteinogenic analog thereof;

L is leucine or a non-proteinogenic analog thereof;

N is asparagine or a non-proteinogenic analog thereof;

$X, X_2, X_3, X_4, X_5, X_6, X_7, X_{11}$ and $X_{16}$ are each a stretch of 1, 2, 3, 4, 5, 6, 7, or 16, respectively proteinogenic amino acids, non-proteinogenic amino acids, D-amino acids, peptidomimetic building blocks or combinations thereof;

K is lysine or a non-proteinogenic analog thereof;

G is glycine or a non-proteinogenic analog thereof;

F is phenylalanine or a non-proteinogenic analog thereof;

E is glutamic acid or a non-proteinogenic analog thereof;

Y is tyrosine or a non-proteinogenic analog thereof; and

V is valine or a non-proteinogenic analog thereof and have the same three-dimensional conformation as naturally occurring CHIPS.

In a further embodiment of the invention the compounds are of the general formula:

$$X_n\text{-}T\text{-}L\text{-}X_5\text{-}R\text{-}L\text{-}R\text{-}N\text{-}Y\text{-}L\text{-}K\text{-}K\text{-}G\text{-}T\text{-}K\text{-}X_2\text{-}A\text{-}X\text{-}F\text{-}E\text{-}K\text{-}X\text{-}V\text{-}I\text{-}L\text{-}X_5\text{-}Y\text{-}X\text{-}T\text{-}$$

$$X_2\text{-}L\text{-}X_3\text{-}L\text{-}X_2\text{-}D\text{-}R\text{-}K\text{-}X_2\text{-}E\text{-}L\text{-}X\text{-}G\text{-}X\text{-}M\text{-}X_2\text{-}T\text{-}Y\text{-}X_2\text{-}K\text{-}K\text{-}G\text{-}E\text{-}X\text{-}K\text{-}X_2\text{-}Y\text{-}V\text{-}$$

$$I\text{-}N\text{-}X_3\text{-}K\text{-}X_5\text{-}Y\text{-}X_m \qquad\qquad (IV)$$

wherein

$X_n$ may or may not be present and is a stretch of proteinogenic amino acids, non-proteinogenic amino acids, D-amino acids, peptidomimetic building blocks or combinations thereof;

T is threonine or a non-proteinogenic analog thereof;

R is arginine or a non-proteinogenic analog thereof;

L is leucine or a non-proteinogenic analog thereof;

I is isoleucine or a non-proteinogenic analog thereof;

M is methionine or a non-proteinogenic analog thereof;

A is alanine or a non-proteinogenic analog thereof;

D is asparagine or a non-proteinogenic analog thereof;

N is asparagine or a non-proteinogenic analog thereof;

K is lysine or a non-proteinogenic analog thereof;

G is glycine or a non-proteinogenic analog thereof;

F is phenylalanine or a non-proteinogenic analog thereof;

E is glutamic acid or a non-proteinogenic analog thereof;

Y is tyrosine or a non-proteinogenic analog thereof; and

V is valine or a non-proteinogenic analog thereof

$X, X_2, X_3$ and $X_5$, are each a stretch of 1, 2, 3 or 5, respectively proteinogenic amino acids, non-proteinogenic amino acids, D-amino acids, peptidomimetic building blocks or combinations thereof;

and have the same three-dimensional conformation as naturally occurring CHIPS.

In formula I, $X_n$ and $X_m$ may each contain 0-100, preferably 1-90, more preferably -75, most preferably 1-59.

In formula II, $X_n$ and $X_m$ may each contain 0-100, preferably 1-50, more preferably 1-25, most preferably 1-15.

In formulas III and IV, $X_n$ and $X_m$ may each contain 0-100, preferably 1-50, more preferably 1-25, most preferably 1-6.

More specific embodiments are CHIPSΔ1, CHIPSΔ17, CHIPSΔ30, lacking 1, 17 and 30 amino acids of the N-terminal domain of naturally occurring CHIPS. Natural CHIPS is not intended to be encompassed by the invention as claimed.

The invention thus relates to compounds that have a backbone in which the same or similar amino acids residues are presented in the same or similar conformation as in CHIPS which allows them to bind to the aspartates on position 10, 15, and 18 (D10, D15, and D18) and the glycine on position 12 (G12) of C5aR. The identification of the amino acid residues of CHIPS that are involved in binding is the basis for the production of a range of compounds that may be very different but that have one thing in common, namely the binding residues in the required conformation. On the basis of this, the skilled person in molecular modeling, will be able to design compounds that meet these requirements and that can thus be used for the treatment of indications involving the C5aR by the use of antibodies or so-called binding bodies, by screening peptide libraries or other methods employing these known sequences as a template.

Presenting the binding residues in the compounds of the invention in the same or a similar conformation as found in naturally occurring CHIPS can be done in various ways, such as by linking the hot spots by means of amino acids, non-proteinogenic analogs, or other molecules that span the same spatial distance.

Alternatively, the information provided from the side of C5aR can be used to screen libraries of compounds for compounds that bind to the C5aR. The invention thus provides a method for the identification of compounds that can bind to the C5aR, comprising

a) provision of a library of candidate compounds,

b) contacting the members of the library with a C5aR related compound that comprises the motif $X_q$-D-X-G-$X_2$-D-$X_2$-D-$X_r$, wherein $X_q$, X, $X_2$ and $X_r$ are stretches of 0-9, 1, 2 and 0-20, respectively, proteinogenic amino acids or non-proteinogenic analogs thereof, D is asparaginic acid and G is glycine, in a conformation that is the same or similar as the conformation found in naturally occurring C5aR,

c) identifying the members of the library that bind to the C5aR related compound. The C5aR related compound can be C5aR itself or compounds that comprise at least the residues that are now found to be involved in binding CHIPS in the naturally occurring conformation.

The above method may further comprise the step of providing the thus identified members for use in prophylaxis or therapy. The invention also relates to the compounds thus identified and their use.

The compounds of the invention that have the C5aR binding residues as found in CHIPS are suitably peptides or polypeptides. All of these peptides may however be used as a starting point for further modification, for example by substitution of one or more of the amino acids with other building blocks.

The peptides having the ability to bind C5aR can be produced by known chemical synthesis. Methods for constructing peptides by synthetic means are known to those skilled in the art. These synthetic peptides, by virtue of sharing primary, secondary and/or tertiary structural and/or conformational characteristics with the stretch of CHIPS that is involved in binding to C5aR will posses an activity in common therewith, meaning C5aR binding properties. Thus, such synthetically produced peptides can be employed as biologically active or immunological substitute for (poly)peptides that have the required C5aR binding ability and consist of proteinogenic amino acids.

The compounds provided herein also include compounds characterized by amino acid sequences into which modifications are naturally provided or deliberately engineered. Modifications in the peptide can be made by those skilled in the art using known conventional techniques. Modifications of interest in the sequence of the compounds may include replacement, insertion or deletion of selected amino acid residues in the coding sequence.

In some cases the potential for use of peptides in drugs may be limited for several reasons. Peptides may for example be too hydrophilic to pass membranes like the cell-membrane and the blood-brain barrier, and may be rapidly excreted from the body by the kidneys and the liver, resulting in a low bio-availability. Furthermore, peptides may suffer from a poor bio-stability and chemical stability since they may be quickly degraded by proteases, e.g. in the gastro-intestinal tract. Also, peptides generally are flexible compounds which can assume thousands of conformations. The bioactive conformation usually is only one of these possibilities, which sometimes might lead to a poor selectivity and affinity for the target receptor, such as C5aR. Finally, the potency of the peptides may not be sufficient for therapeutical purposes.

As a result of the above described drawbacks, peptides are sometimes mainly used as sources for designing other drugs, and not as actual drugs themselves. In such case it is desirable to develop compounds in which these drawbacks have been reduced. Alternatives for peptides are the so-called peptidomimetics. Peptidomimetics based on the compounds of the present invention are also part of this application. In that case, one or more of the amino acids are substituted with peptidomimetic building blocks.

Various definitions for peptidomimetics have been formulated in literature. Among others, peptidomimetics have been described as "chemical structures designed to convert the information contained in peptides into small non-peptide structures", "molecules that mimic the biological activity of peptides but no longer contain any peptide bonds", "structures which serve as appropriate substitutes for peptides in interactions with receptors and enzymes" and as "chemical Trojan horses".

In general, peptidomimetics can be classified into two categories. The first consists of compounds with non-peptidelike structures, often scaffolds onto which pharmacophoric groups have been attached. Thus, they are low molecular-weight compounds and bear no structural resemblance to the native peptides, resulting in an increased stability towards proteolytic enzymes.

The second main class of peptidomimetics consists of compounds of a modular construction comparable to that of peptides, i.e. oligomeric peptidomimetics. These compounds can be obtained by modification of either the peptide side chains or the peptide backbone. Peptidomimetics of the latter category can be considered to be derived of peptides by replacement of the amide bond with other moieties. As a result, the compounds are expected to be less sensitive to degradation by proteases. Modification of the amide bond also influences other characteristics such as lipophilicity, hydrogen bonding capacity and conformational flexibility, which in favourable cases may result in an overall improved pharmacological and/or pharmaceutical profile of the compound.

Oligomeric peptidomimetics can in principle be prepared starting from monomeric building blocks in repeating cycles of reaction steps. Therefore, these compounds may be suitable for automated synthesis analogous to the well-established preparation of peptides in peptide synthesizers. Another application of the monomeric building blocks lies in the preparation of peptide/peptidomimetic hybrids, combining natural amino acids and peptidomimetic building blocks to give products in which only some of the amide bonds have been replaced. This may result in compounds which differ sufficiently from the native peptide to obtain an increased biostability, but still possess enough resemblance to the original structure to retain the biological activity.

Suitable peptidomimetic building blocks for use in the invention are amide bond surrogates, such as the oligo-β-peptides (Juaristi, E. Enantioselective Synthesis of b-Amino Acids; Wiley-VCH: New York, **1996**), vinylogous peptides (Hagihari, M. et al., J. Am. Chem. Soc. **1992,** 114, 10672-10674), peptoids (Simon, R.J. et al., Proc. Natl. Acad. Sci. USA **1992,** 89, 9367-9371; Zuckermann, R.N. et al., J. Med. Chem. **1994,** 37, 2678-2685; Kruijtzer, J.A.W. & Liskamp, R.M.J. Tetrahedron Lett. **1995,** 36, 6969-6972); Kruijtzer, J.A.W. Thesis; Utrecht University, **1996;** Kruijtzer, J.A.W. et al., Chem. Eur. J. **1998,** *4*, 1570-1580), oligosulfones (Sommerfield, T. & Seebach, D. Angew. Chem., Int. Ed. Eng. **1995,** 34, 553-554), phosphodiesters (Lin, P.S.; Ganesan, A. Bioorg. Med. Chem. Lett. **1998,** 8, 511-514), oligosulfonamides (Moree, W.J. et al., Tetrahedron Lett. **1991,** 32, 409-412; Moree, W.J. et al., Tetrahedron Lett. **1992,** 33, 6389-6392; Moree, W.J. et al., Tetrahedron **1993,** 49, 1133-1150; Moree, W.J. Thesis; Leiden University, **1994;** Moree, W.J. et al., J. Org. Chem. **1995,** 60, 5157-5169; de Bont, D.B.A. et al., Bioorg. Med. Chem. Lett. **1996,** 6, 3035-3040; de Bont, D.B.A. et al., Bioorg. Med. Chem. **1996,** 4, 667-672; Löwik, D.W.P.M. Thesis; Utrecht University, **1998**), peptoid sulfonamides (van Ameijde, J. & Liskamp, R.M.J. Tetrahedron Lett. **2000,** 41, 1103-1106), vinylogous sulfonamides (Gennari, C. et al., Eur. J. Org. Chem. **1998,** 2437-2449), azatides (or hydrazinopeptides) (Han, H. & Janda, K.D. *J. Am. Chem. Soc.* **1996,** *118*, 2539-2544), oligocarbamates (Paikoff, S.J. et al., Tetrahedron Lett. **1996,** 37, 5653-5656; Cho, C.Y. et al., Science **1993,** 261, 1303-1305), ureapeptoids (Kruijtzer, J.A.W. et al., Tetrahedron Lett. **1997,** 38, 5335-5338; Wilson, M.E. & Nowick, J.S. Tetrahedron Lett. **1998,** 39, 6613-6616) and oligopyrrolinones (Smith III, A. B. et al., J. Am. Chem. Soc. **1992,** 114, 10672-10674).

The vinylogous peptides and oligopyrrolinones have been developed in order to be able to form secondary structures (β-strand conformations) similar to those of peptides, or mimic secondary structures of peptides. All these oligomeric peptidomimetics are expected to be resistant to proteases and can be assembled in high-yielding coupling reactions from optically active monomers (except the peptoids).

Peptidosulfonamides are composed of α- or β-substituted amino ethane sulfonamides containing one or more sulfonamide transition-state isosteres, as an analog of the hydrolysis of the amide bond. Peptide analogs containing a transition-state analog of the hydrolysis of the amide bond have found a widespread use in the development of protease inhibitor e.g. HIV-protease inhibitors.

Another approach to develop oligomeric peptidomimetics is to completely modify the peptide backbone by replacement of all amide bonds by nonhydrolyzable surrogates e.g. carbamate, sulfone, urea and sulfonamide groups. Such oligomeric peptidomimetics may have an increased metabolic stability. Recently, an amide-based alternative oligomeric peptidomimetics has been designed viz. N-substituted Glycine-oligopeptides, the so-called peptoids. Peptoids are characterized by the presence of the amino acid side chain on the amide nitrogen as opposed to being present on the α-C-atom in a peptide, which leads to an increased metabolic stability, as well as removal of the backbone chirality. The absence of the chiral α-C atom can be considered as an advantage because spatial restrictions which are present in peptides do not exist when dealing with peptoids. Furthermore, the space between the side chain and the carbonyl group in a peptoid is identical to that in a peptide. Despite the differences between peptides and peptoids, they have been shown to give rise to biologically active compounds.

Translation of a peptide chain into a peptoid peptidomimetic may result in either a peptoid (direct-translation) or a retropeptoid (retro-sequence). In the latter category the relative orientation of the carbonyl groups to the side chains is maintained leading to a better resemblance to the parent peptide.

Review articles about peptidomimetics that are incorporated herein by reference are:

Adang, A.E.P. et al.; Recl. Trav. Chim. Pays-Bas 1994, 113, 63-78; Giannis, A. & Kolter, T. Angew. Chem. Int. Ed. Engl. 1993, 32,1244-1267; Moos, W.H. et al., Annu. Rep. Med. Chem. 1993, 28, 315-324; Gallop, M.A. et al., J. Med. Chem. 1994, 37, 1233-1251; Olson, G.L. et al., J. Med. Chem. 1993, 36, 3039-30304; Liskamp, R.M.J. Recl. Trav. Chim. Pays-Bas 1994, 113, 1-19; Liskamp, R.M.J. Angew. Chem. Int. Ed. Engl. 1994, 33, 305-307; Gante, J. Angew. Chem. Int. Ed. Engl. 1994, 33, 1699-1720; Gordon, E.M. et al., Med. Chem. 1994, 37, 1385-1401; and Liskamp, R.M.J. Angew. Chem. Int. Ed. Engl. 1994, 33, 633-636.

The invention thus furthermore relates to compounds that are not peptides or polypeptides themselves but have a structure and function similar to those of the compounds of this invention. Examples of such molecules are the above described peptidomimetics, but also compounds in which one or more of proteinogenic amino acids are replaced by non-proteinogenic amino acids or D-amino acids. When reference is made in this application to compounds, it is intended to include also such other compounds that have a similar or the same structure and function and as a consequence a similar or the same biological activity as the original peptide compounds.

More in particular substitutions can be made with non-proteinogenic amino acids selected from the group consisting of 2-naphtylalanine (Nal(2)), β-cyclohexylalanine (Cha), p-amino-phenylalanine ((Phe(p-NH$_2$), p-benzoyl-phenylalanine (Bpa), ornithine (Orn), norleucine (Nle), 4-fluoro-phenylalanine (Phe(p-F)), 4-chloro-phenylalanine (Phe(p-Cl)), 4-bromo-phenylalanine (Phe(p-Br)), 4-iodo-phenylalanine (Phe(p-I)), 4-methyl-phenylalanine (Phe(p-Me)), 4-methoxy-phenylalanine (Tyr(Me)), 4-nitro-phenylalanine (Phe(p-N02)).

Suitable D-amino acids for substituting the proteinogenic amino acids in the compounds of the invention are for example those that are selected from the group consisting of D-phenylalanine, D-alanine, D-arginine, D-asparagine, D-aspartic acid, D-cysteine, D-glutamic acid, D-glutamine, D-histidine, D-isoleucine, D-leucine, D-lysine, D-methionine, D-proline, D-serine, D-threonine, D-tryptophan, D-tyrosine, D-valine, D-2-naphtylalanine (D-Nal(2)), β-cyclohexyl-D-alanine (D-Cha), 4-amino-D-phenylalanine (D-Phe(p-NH$_2$)), p-benzoyl-D-phenylalanine (D-Bpa), D-Ornithine (D-Orn), D-Norleucine (D-Nle), 4-fluoro-D-phenylalanine (D-Phe(p-F)), 4-chloro-D-phenylalanine (D-Phe(p-Cl)), 4-bromo-D-phenylalanine (D-Phe(p-Br)), 4-iodo-D-phenylalanine (D-Phe(pI)), 4-methyl-D-phenylalanine (D-Phe(p-Me)), 4-methoxy-D-phenylalanine (D-Tyr(Me)), 4-nitro-D-phenylalanine (D-Phe(p-NO2)).

One or more of the proteinogenic amino acids in the compounds of the invention can be replaced by peptoid building blocks, e.g. selected from the group consisting of N-substituted glycines, such as N-benzylglycine (NPhe), N-methylglycine (NAla), N-(3-guanidinopropyl)glycine (NArg), N-(Carboxymethyl)glycine (NAsp), N-(carbamylmethyl)glycine (NAsn), N-(thioethyl)-glycine (NhCys), N-(2-carboxyethyl)glycine (NGlu), N-(2-carbamylethyl)glycine (NGln), N-(imidazolylethyl)glycine (NhHis), N-(1-methylpropyl)glycine (NIle), N-(2-methylpropyl)glycine (NLeu), N-(4-aminobutyl)glycine (NLys), N-(2-methylthioethyl)glycine (NMet), N-(hydroxyethyl)glycine (NhSer), N-(2-hydroxypropyl)glycine (NhThr), N-(3-indolylmethyl)glycine (NTrp), N-(p-hydroxyphenmethyl)-glycine (NTyr), N-(1-methylethyl)glycine (NVal).

All compounds of the invention may also be in cyclic form. A cyclic compound may have improved potency, stability, rigidity and/or other pharmaceutical and/or pharmacological characteristics.

According to another aspect thereof the invention relates to antibodies or derivatives thereof against C5aR or a C5aR related compound that comprises the motif $X_q$-D-X-G-$X_2$-D-$X_2$-D-$X_r$, wherein $X_q$, X, $X_2$ and $X_r$ are stretches of 0-9, 1, 2 and 0-20. Derivatives as used herein are well-known compounds that have the specificity of antibodies but are not complete antibodies anymore. Examples of derivatives are well known in the art and comprise scFv, Fab fragments, chimeric antibodies, bifunctional antibodies and others.

The functional activity of compounds of the invention can be assayed by various methods. This C5aR binding activity of a compound can be measured by its ability to prevent the binding of fluorescent-C5a (such as FITC-C5a) to neutrophils as determined by flow cytometry.

The activity of a compound of the invention is also measured by its ability to prevent migration of neutrophils towards C5a as determined by chemotaxis assays, such as the Transwell system. Furthermore, an assay based on the ability of chemokines, including C5a, to initiate a rapid and transient rise in intracellular calcium concentration can be employed to screen for biological activity of the compound of the invention. Alternatively, an assay based on the ability of chemokines, including C5a, to initiate an excretion of e.g. elastase in cytochalasin B-stimulated neutrophils, can be used to screen for biological activity of the compound of the invention. Various other assays known in the art can be used, including but not limited to the use of various calcium specific fluorescent probes in combination with flow cytometry or fluorometry, or microphysiometry. As cells for the screening of biological activity by either method, e.g. freshly isolated neutrophils can be used or cells transfected with C5aR, wild type or mutated forms of this receptor.

The compounds of the invention can be used the prophylaxis or treatment of indications involving the C5a-receptor (C5aR) on neutrophils, monocytes and endothelial cells. Such indication may involve acute or chronic inflammation reactions and can be selected from the group listed in Table 4. Table 4 is not intended to be limiting to the possible use of the compounds of the invention. Use of the compounds in future indications is also part of this invention.

The invention further relates to use of the compounds in the prophylaxis or treatment of indications involving C5aR on cells other than neutrophils, monocytes and endothelial cells. The other cells can be lymphocytes, dendritic cells, eosinophils, basophils, macrophages, microglia cells, astrocytes, Kupfer cells, hepatocytes and epithelial cells.

The compounds of the invention can also be used in prophylactic or therapeutic vaccines for infections caused by CHIPS-producing bacteria, such as *Staphylococcus aureus.*

The compounds of the invention can also be used for the preparation of a coating composition for use on surfaces of medical devices that are introduced into the human body through the skin, or placed in the body during surgical procedures. Such surface may be the surface is the surface of a catheter tip. The composition is suitably a slow-release composition.

The invention relates to the compounds *per se* and to the various uses of the compounds as described above. Furthermore, the invention provides therapeutic and prophylactic compositions comprising a suitable excipient and one or more compounds as claimed.

The invention according to a further aspect thereof relates to methods for prophylaxis or treatment of a subject suffering from indications involving C5aR on neutrophils, monocytes and endothelial cells comprising administering a prophylactically or therapeutically effective amount of one or more of the compounds as claimed. The indications to be treated are the same as described above.

In another embodiment thereof, the invention relates to methods for prophylaxis or treatment of a subject suffering from indications involving C5aR on cells other than neutrophils, monocytes and endothelial cells comprising administering a prophylactically or therapeutically effective amount of one or more of the compounds as claimed. The other cells are lymphocytes, dendritic cells, eosinophils, basophils, macrophages, microglia cells, astrocytes, Kupfer cells, hepatocytes and epithelial cells.

A further embodiment of the invention relates to methods for the prophylactic or therapeutic treatment of a subject against infections with CHIPS-producing bacteria comprising the administration of a prophylactically or therapeutically effective amount of one or more compounds as claimed.

Twenty amino acids are encoded by the standard genetic code and are called proteinogenic (protein building). Over 500 other amino acids have been found in nature. These other amino acids are called herein "non-proteinogenic amino acids".

All of the amino acids in proteins exhibit the same absolute steric configuration as L-glyceraldehyde. Therefore, they are all L-amino acids. D-amino acids are never found in proteins, although they exist in nature. Herein they are called "D-amino acids".

A peptidomimetic is a compound containing non-peptidic structural elements that is capable of mimicking or antagonizing the biological action(s) of a natural parent peptide. A peptidomimetic does no longer have classical peptide characteristics such as peptidic bonds. The components of the peptidomimetic are called herein "peptidomimetic building blocks".

All non-proteinogenic variants of a particular amino acid are called herein "non-proteinogenic analogs thereof".

The present invention will be further illustrated in the examples that follow and that are in no way intended to be limiting to this invention. In the examples reference is made to the following figures:

> **Figure 1** shows the binding of CHIPS-FITC to the C5aR N-terminus, containing diverse point mutations and deletions, expressed on HEK-293 cells using the pDISPLAY vector.
>
> **Figure 2A** shows the binding of CHIPS-FITC to the whole C5aR, containing diverse point mutations and deletions in its N-terminus, expressed on HEK-293 cells using the pcDNA3.1 vector.
>
> **Figure 2B** shows the binding of CHIPS-FITC to the whole C5aR and C5aR chimeras, in which the N-terminus and the extra-cellular loops are exchanged for the corresponding regions of other G protein-coupled receptors. In most of the chimeras also an additional D10A mutation was constructed. All receptors were expressed on HEK-293 cells using the pcDNA3.1 vector.
>
> **Figure 3** shows the inhibition of CHIPS-FITC binding to the wild type C5aR expressed on U937 cells by a SO$_4$-C5aR-N-terminal peptide, comprising amino acids 7 to 28.
>
> **Figure 4** shows the potencies of WT-CHIPS and CHIPS31-121 to inhibit the C5a-induced calcium mobilization in human neutrophils.
>
> **Figure 5A+B** show the inhibition of the binding of anti-C5aR monoclonal antibody to the C5aR, expressed on U937 cells, by CHIPS31-121 and indicated mutants of CHIPS31-121.

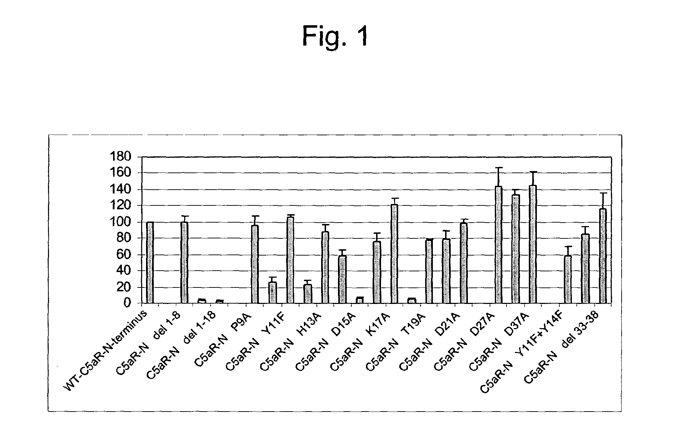

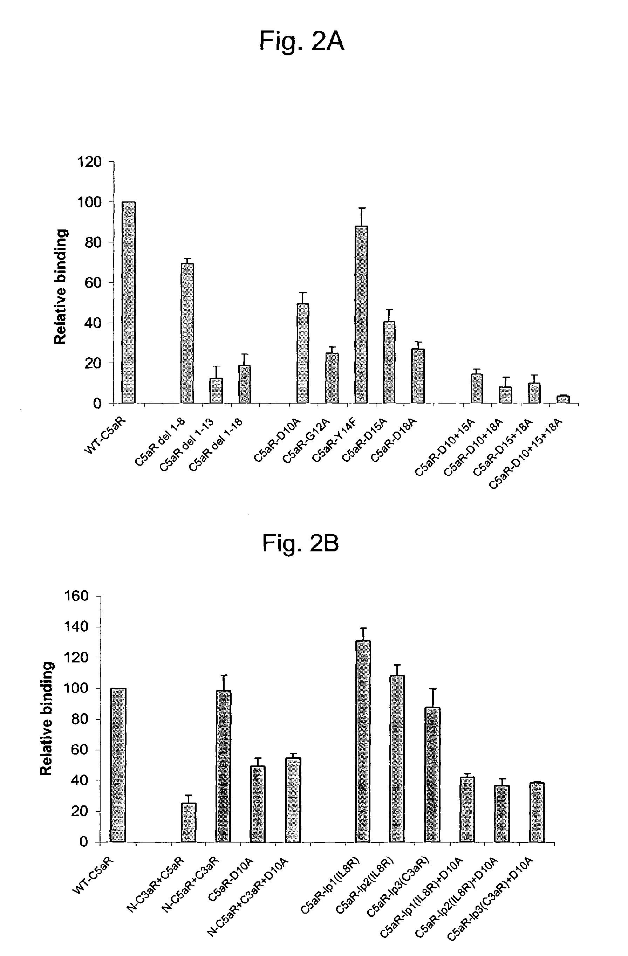

Table 1 shows all amino acids of the C5aR that are involved in the binding to wild-type CHIPS (WT-CHIPS), as obtained by testing all C5aR mutants and C5aR chimeras for WT-CHIPS binding, as depicted in figures 1 and 2.

**Table 2** shows all amino acids of CHIPS31-121 that are or might be involved in the binding of CHIPS31-121 to the C5aR, as obtained by titration of $SO_4$-C5AR pep7-28 to $^{15}N$-labeled CHIPS31-121 in a $^{15}N$-1H-HSQC NMR experiment.

**Table 3** shows the $IC_{50}$ values of diverse CHIPS31-121 mutants, in which single amino acids were substituted into alanines (unless otherwise stated). $IC_{50}$ determinations were done using the experiments shown in figure 6A+B.

**Table 4** lists diseases and disease states caused by inflammatory reactions, involving complement activation and/or neutrophil and/or monocyte involvement. Support for the therapeutical usefulness of the poly)peptides of the invention for treatment of these diseases can be found in the following references:

For *ARDS:* Demling RH (1995). The modern version of adult respiratory distress syndrome. Ann. Rev. Med. 46:193-202; and Fujishima S, Aikawa N (1995) Neutrophil-mediated tissue injury and its modulation. Intensive Care Med 21:277-285;

For *severe infections (meningitis):* Tunkel AR and Scheld WM (1993). Pathogenesis and pathophysiology of bacterial meningitis. Clin. Microbiol. Rev. 6:118. For injury after ischaemia/reperfusion: Helier T, et al. (1999). Selection of a C5a receptor antagonist from phage libraries attenuating the inflammatory response in immune complex disease and ischemia/reperfusion injury. J. Immunol. 163:985-994.

For *rheumatoid arthritis:* Edwards SW and Hallett MB (1997). Seeing the wood for the trees: the forgotten role of neutrophils in rheumatoid arthritis. Immunology Today 18: 320-324; and Pillinger MH, Abramson SB (1995). The neutrophil in rheumatoid arthritis. Rheum. Dis. Clin. North Am. 1995 21:691-714.

For *myocardial infarction:* Byrne JG, Smith WJ, Murphy MP, Couper GS, Appleyard RF, Cohn LH (1992). Complete prevention of myocardial stunning, contracture, low-reflow, and edema after heart transplantation by blocking neutrophil adhesion molecules during reperfusion. J. Thorac. Cardiovasc. Surg. 104:1589-96.

For *COPD:* Cox G (1998). The role of neutrophils in inflammation. Can. Respir. J. 5 Suppl A:37A-40A; and Hiemstra PS, van Wetering S, Stolk J (1998). Neutrophil serine proteinases and defensins in chronic obstructive pulmonary disease: effects on pulmonary epithelium. Eur. Respir. J. 12:1200-1208.

For *stroke:* Barone FC, Feuerstein GZ (1999). Inflammatory mediators and stroke: new opportunities for novel therapeutics. J. Cereb. Blood Flow Metab. 19:819-834; and Jean WC, Spellman SR, Nussbaum ES, Low WC (1998). Reperfusion injury after focal cerebral ischemia: the role of inflammation and the therapeutic horizon. Neurosurgery 43:1382-1396.

For *meningitis:* Tuomanen EI (1996). Molecular and cellular mechanisms of pneumococcal meningitis. Ann. N. Y. Acad. Sci. 797:42 52.

For all *directly complement related diseases:* Adapted from: A. Sahu and J.D. Lambris Immunopharmacology 49 (2000) 133-148.

## EXAMPLES

## EXAMPLE 1

Identification of the CHIPS binding-site within the C5aR

### *MATERIALS AND METHODS*

*1.1 Cloning of the whole C5aR and the C5aR N-terminus*

The DNA sequence encoding the complete human C5aR was amplified by PCR using primers whose sequences were modified to introduce an N-terminal FLAG epitope tag (DYKDDDDK) downstream the first methionine (start codon) of the C5aR open reading frame (ORF) and restriction sites (EcoRI and XbaI) permitting the cloning of the FLAG-tagged C5aR sequence in the expressing plasmid pcDNA3.1 (Invitrogen, Paisley, UK). The fragment was inserted in the appropriate orientation, permitting the expression of the FLAG-tagged C5aR by the CMV promoter. The cloned FLAG-tagged C5aR sequence was verified by sequence analysis. The FLAG-tag allows detection of the C5aR by monoclonal antibody (mAb) M2 (Sigma Chemical Co., St.Louis, MO, USA).

The cloning of the DNA sequence encoding the first 38 amino acids of the human C5aR (which equals the C5aR N-terminus) was performed by PCR using primers whose sequences were modified to introduce restriction sites (AccI and BglII) permitting the cloning of the C5aR N-terminal sequence in the expressing plasmid pDISPLAY (Invitrogen, Paisley, UK). The fragment was inserted in the appropriate orientation, permitting the expression of the C5aR N-terminus by the CMV promoter. A murine Ig kappa-chain V-J2-C signal peptide sequence present upstream of the C5aR N-terminal sequence enables targeting of the C5aR N-terminal protein to the secretory pathway of eukaryotic cells. The PDGF receptor transmembrane domain present downstream of the C5aR N-terminal sequence anchors the C5aR N-terminal protein to the plasma membrane of eukaryotic cells for display. A hemagglutinin A epitope tag, present upstream of the C5aR N-terminal sequence and downstream of the signal peptide, allows detection of the pDISPLAY C5aR N-terminal protein by mAb 12CA5 (Roche, Molecular Biochemicals, Mannheim, Germany). The cloned C5aR N-terminal sequence was verified by sequence analysis.

*1.2 CHIPS production*

Chromosomal DNA of *S. aureus,* coding for WT-CHIPS (CHIPS 1-121), was amplified by PCR with Thermal Ace DNA polymerase (Invitrogen, Paisley, UK) using the primers: 5' TTTACTTTTGAACCGTTTCCTAC for CHIPS 1-121 combined with 3' CGTCCTGAATTCTTAGTATGCATATTCATTAG (underlined sequence represents an EcoRI site). The amplification reaction was performed using chromosomal DNA of *S. aureus* Newman as a template, which was isolated with a high pure PCR template preparation kit (Roche Molecular Biochemicals, Mannheim, Germany). The pRSET expression vector (Invitrogen), providing a N-terminal histidine tag, was digested with BamHI and treated with S1 nuclease (Roche Diagnostics; 100 U/ml, 30 min 37°C) to provide a blunt ended vector exactly after the DNA sequence encoding the enterokinase cleavage site.
Subsequently, the vector was digested with EcoRI and ligated with the EcoRI-digested PCR products. Plasmids were propagated in TOP10F' *E. coli.* For expression, BL21(DE3) *E. coli* were transformed, 1 colony was grown overday in 2 ml LB, containing 50 µg/ml carbenicillin (Sigma) and subsequently overnight (1:1000). The next morning, cells were pelleted and resuspended into fresh LB, containing 50 µg/ml carbenicillin to obtain an $OD_{660}$ of 0.1. The cultures were grown to an $OD_{660}$ of 0.8 and induced with 1 mM IPTG for 2.5 h at 37°C. Then, the cells were pelleted and resuspended in 20 mM sodium phosphate buffer, pH 7.8, containing 500 mM NaCl and kept at -20°C until use.
For isolation of CHIPS1-121 or CHIPS31-121, the cells were treated for 15 min with 100 µg/ml egg white lysozym (Sigma) on ice. For further cell lysis, the bacteria were sonicated, frozen in liquid $N_2$ and thawed in a 37°C water bath for a total of four cycles.
Thereafter, RNase and DNase (5 µg/ml) was added for 30 min on ice. Subsequently, the lysate was centrifuged at 3000 g for 30' at 4°C, filtered through a 0.45 µm filter, diluted 1:1 with cold phosphate buffer pH 7.8 and run through a charged Nickel column (Invitrogen) at 0.2 ml/min. The column was washed with phosphate buffers pH 7.8, pH 6.0, and pH 5.3, respectively. The column is eluted with PBS/50 mM EDTA. The histidine tag was removed by enterokinase cleavage (1 U/ml protein) for 4 h at room temperature.

*1.3 FITC labeling of purified CHIPS protein*

Purified CHIPS (500 µg/ml protein) is incubated with 1/10th volume of 1 mg/ml FITC (Fluorescein Isothiocyanate, Isomer I; Sigma) in a 1 M Sodium carbonate buffer pH 9.6 for 1 hour at room temperature. FITC- labeled CHIPS is separated from free FITC by passing the mixture over a desalting column (Pharmacia, Fast Desalting HR 10/10) and monitoring the eluate for $OD_{280}$ and fluorescence by an on-line coupled fluorometer (Perkin Elmer). Fractions with high $OD_{280}$ and fluorescence were pooled and analyzed for protein content with the Micro BCA protein assay (Pierce). CHIPS-FITC is stored in small aliquots at -20°C.

1.4 *Expression of* the *C5aR and CSaR N-terminus*

The whole C5aR was expressed by transfection of the pcDNA3.1 plasmid, containing the ORF of the C5aR, in HEK293 cells. Therefore, HEK293 cells, maintained in MEM (Gibco Invitrogen Corporation), containing 0.1 mM non essential amino acids, 1 mM sodiumpyruvate, 10 µg/ml gentamycin and 10% FCS, were transfected in a 6 well plate with 1 µg of DNA using lipofectamine 2000 (5 µl), according to the manufacturer's descriptions (Invitrogen).
Two to three days after transfection the cells were collected with PBS/trypsin, washed with RPMI/0.05% human serum albumin (HSA; CLB, Amsterdam, The Neterlands) and incubated with the 10 µg/ml anti-FLAG M2 mAb and 3 µg/ml FITC-labeled CHIPS, and subsequently with 2 µg/ml APC-labeled goat-anti-mouse Igs (PharMingen, San Diego, CA, USA). Cells were washed and assayed on a flow cytometer, after addition of propidium jodide, for binding of CHIPS-FITC to the C5aR N-terminal protein-expressing(APC-positive) and propidium jodide-negative cells (living) cells.
For expressing of the C5aR N-terminus, the pDISPLAY plasmid, containing the C5aR N-terminal sequence was transfected in HEK293 cells as described above, except that for detection of CHIPS-FITC binding, the cells were incubated with 5 µg/ml anti-HA tag 12CA5 mAb in stead of the anti-FLAG M2 mAb.

1.5 *Cloning and expression of C5aR- or C5aR N-terminus-mutants and C5aR chimeras*

Site-directed mutagenesis was performed on the C5aR-and/or C5aR N-terminus by overlap extension PCR (Ho SN et al., 1989, Gene 77;51). Mutagenesis was performed on all amino acids from the C5aR N-terminus ranging from amino acid proline 9 (denominated as P9) up to aspartate 21 (denominated as D21). The complete amino acid sequence of the C5aR N-terminus is depicted in table 1. All amino acids were mutated into alanines (A; denominated as P9A, D10A, etc.), as single mutations or in combinations. Only the tyrosines (Y) 11 and 14 were changed into phenylalanines (F; denominated as Y11F etc.) instead of into alanines. Other mutants were deleted in the N-terminal 8, 13, or 18 amino acids, denominated as del-8, del-13, del-18. Also the following C5aR chimeras were constructed by overlap extention PCR:

* N-C3aR+C5aR: N-terminus of the C5aR (corresponding to C5aR amino acids 1-37) was exchanged for the N-terminus of the C3aR (corresponding to C3aR amino acids 1-23)
* N-C5aR+C3aR: N-terminus of the C3aR (corresponding to C3aR amino acids 1-23) was exchanged for the N-terminus of the C5aR (corresponding to C5aR amino acids 1-37)
* C5aR-lp1(IL8R): extra-cellular loop 1 of the C5aR (corresponding to C5aR amino acids 90-117) was exchanged for the extra-cellular loop 1 of the IL8R (corresponding to IL8R amino acids 93-118).
* C5aR-lp2(IL8R): extra-cellular loop 2 of the C5aR (corresponding to C5aR amino acids 172-211) was exchanged for the extra-cellular loop 2 of the IL8R (corresponding to IL8R amino acids 172-211).
* C5aR-lp3(C3aR): extra-cellular loop 3 of the C5aR (corresponding to C5aR amino acids 265-280) was exchanged for the extra-cellular loop 3 of the C3aR (corresponding to C3aR amino acids 400-415).

In all above described C5aR chimeras, except for the N-C3aR+C5aR, also an extra D10A mutation was constructed. PCR fragments of the mutated complete C5aR and C5aR chimeras were cloned into the pcDNA3.1 plasmid, as described in 1.1. PCR fragments of the mutated C5aR N-terminus were cloned into the pDISPLAY plasmid, as described in 1.1.

*RESULTS*

**Figure 1** shows the binding of CHIPS-FITC to the C5aR N-terminus, containing diverse point mutations and deletions, expressed on HEK-293 cells using the pDISPLAY vector. It follows that binding is almost completely abolished by mutations in positions 15 and 18 and deletions of amino acids 1-13 and up.

**Figure 2A** shows the binding of CHIPS-FITC to the whole C5aR, containing diverse point mutations and deletions in its N-terminus, expressed on HEK-293 cells using the pcDNA3.1 vector. It follows that mutations of the three aspartates in positions 10, 15 and 18 most significantly reduces binding of CHIPS-FITC to the whole C5aR.

**Figure 2B** shows the binding of CHIPS-FITC to the whole C5aR and C5aR chimeras, in which the N-terminus and the extra-cellular loops are exchanged for the corresponding regions of other G protein-coupled receptors. In most of the chimeras also an additional D10A mutation was constructed. All receptors were expressed on HEK-293 cells using the pcDNA3.1 vector. **Figure 2B** shows that none of the extracellular loops of the C5aR are involved in the binding to CHIPS. Thus, CHIPS only binds to the C5aR N-terminus.

**Table 1** shows all amino acids of the C5aR that are involved in the binding to WT-CHIPS, as obtained by testing all C5aR mutants and C5aR chimeras for WT-CHIPS binding, as depicted in **figures 1 and 2**. The aspartates in positions 10, 15 and 18 and the glycine in position 12 were found to be involved. There is no involvement of amino acids 38-350 found.

**EXAMPLE 2**

Effect of the sulfonated C5aR N-terminal peptide 7-28 on the CHIPS-FITC binding to the C5aR

***MATERIALS AND METHODS***

*Effect of the $SO_4$-C5aR pep7-28 on the CHIPS-FITC binding to the C5aR*

CHIPS-FITC (0 to 1 μg/ml) was preincubated with 200 μM of $SO_4$-C5aR pep7-28 for 30 min at 4°C. Then, U937 cells ($5 \times 10^6$ cells/ml), expressing the wild type C5aR, were added and incubated for another 30 min at 4°C. Subsequently, the binding of CHIPS-FITC to the U937 cells was analyzed on a flow cytometer.

***RESULTS***

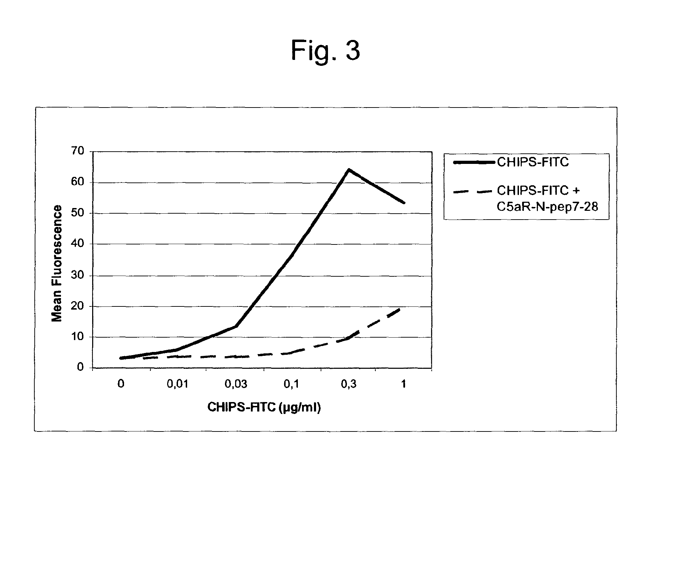

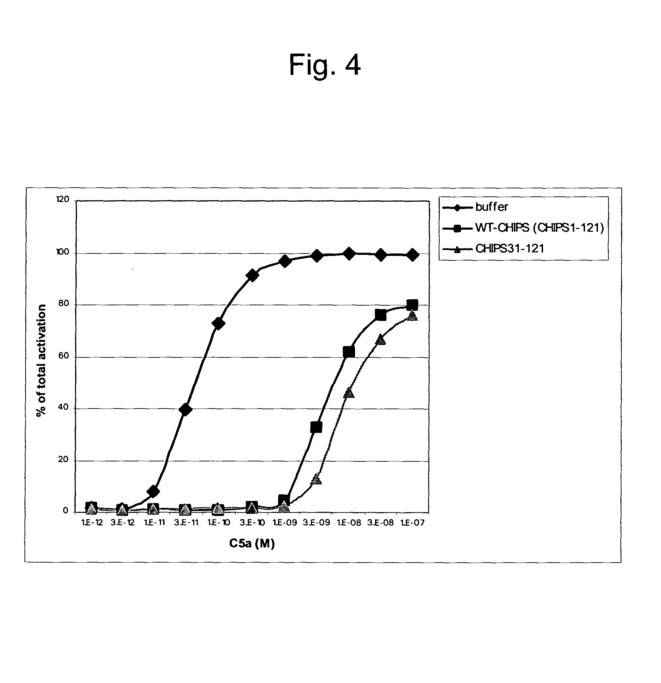

**Figure 3** shows the inhibition of CHIPS-FITC binding to the wild type C5aR expressed on U937 cells by $SO_4$-C5aR pep7-28. One can conclude from these results that only a part of the C5aR N-terminus (amino acids 7 to 28), synthesized as a sulfated peptide (both tyrosines present in this peptide are sulfated, as is also the case in the natural C5aR), is able to bind to CHIPS and prevent it from binding to the C5aR expressed on the U937 cells.

**EXAMPLE 3**

Comparison in C5a-inhibiting activity of WT-CHIPS (CHIPSI-121) and CHIPS31-121

***MATERIALS AND METHODS***

3.1 *Expression and purification of CHIPS31-121*

CHIPS31-121 was cloned, expressed, and purified as described in section 1.2 CHIPS production, using the primers:

$$5' \text{ AATAGTGGTCTTCCTACAAC}$$

and

$$3' \text{ CGTCCT}\underline{\text{GAATTC}}\text{TTAGTATGCATATTCATTAG}$$

(underlined sequence represents an EcoRI site).

3.2 *Intracellular calcium mobilization in neutrophils*

To measure the effect of WT-CHIPS (CHIPS1-121) and CHIPS31-121 on the C5a-induced intracellular calcium mobilization, neutrophils were loaded with 2 $\mu$M Fluo-3AM in RPMI/0.05% HSA for 20 minutes at room temperature under constant agitation, washed twice with buffer and suspended to 1x10$^6$ cells/ml in RPMI/0.05% HSA. Subsequently, the cells were preincubated with or without 30 nM of WT-CHIPS or CHIPS31-121 for 15 min at room temperature. Each sample of cells was first measured for about 10 seconds to determine the basal fluorescence level. Next, concentrated C5a (concentration range C5a used: 10$^{-12}$ M to 10$^{-7}$ M) was added and rapidly placed back in the sample holder to continue the measurement. Cells were analyzed in a FACScan or Calibur flow cytometer gated on forward and side scatter to exclude dead cells and debris.

***RESULTS***

**Figure 4** shows the potencies of WT-CHIPS and CHIPS31-121 to inhibit the C5a-induced calcium mobilization in human neutrophils. It follows that CHIPS31-121 is as potent or even more potent than WT-CHIPS in inhibiting the C5a-induced calcium mobilization. Thus for the C5a-inhibiting potency the first 30 amino acids of WT-CHIPS are of no importance.

**EXAMPLE 4**

Amino acids of CHIPS31-121 involved in the binding of CHIPS to the C5aR

***MATERIALS AND METHODS***

4.1 *Production of uniformly $^{15}N$-labeled and $^{13}C/^{15}N$-labeled CHIPS31-121*

$^{15}N$-labeled and $^{13}CP^{15}N$-labeled CHIPS31-121 (CHIPS, lacking the first 30 amino acids) was cloned and expressed, as described in section 1.2 CHIPS production, using the primers:

$$5' \text{ AATAGTGGTCTTCCTACAAC}$$

and

3' CGTCCT<u>GAATTC</u>TTAGTATGCATATTCATTAG

(underlined sequence represents an EcoRI site).

For $^{15}$N-labeling of CHIPS31-121, BL21(DE3) *E. coli,* transformed with the pRSET/CHIPS31-121 plasmid, were not grown in LB medium, but in M9 minimal medium, containing per litre: 6 g $Na_2HPO_4$, 3 g $KH_2PO_4$, 0.5 g NaCl, 1 g [15]$NH_4Cl$, 2 ml 1M $MgSO_4$, 10 μl $CaCl_2$, 1 ml 0.01 M $FeSO_4$, 10 ml 20% glucose, 1 ml 1000x vitamin solution (1000x vitamin solution contains per litre: 0.4 g Choline chloride, 0.5 g Folic acid, 0.5 g Panthothenic acid, 0.5 g Nicotinamide, 1.0 g Myo-inositol, 0.5 g Pyridoxal HCl 0.5 g Thiamine HCl 0.05 g Riboflavin, and 1 g Biotin), and 1 ml 1000X micronutrients (1000X micronutrients contains per litre: $3x10^{-6}$ M Ammonium molybdate, $4x10^{-4}$ M $H_3BO_3$, $3x10^{-5}$ M $CoCl_2$, $1x10^{-5}$ M CuSO4, $8x10^{-5}$ M $MnCl_2$, and $1x10^{-5}$ M $ZnSO_4$) .

For $^{13}$C/$^{15}$N-labeling of CHIPS31-121, 1 g [15]$NH_4Cl$ and 2 g $^{13}$C-glucose was used per litre M9 minimal medium. Purified CHIPS31-121 samples were dissolved to concentrations of $1^{-2}$ mM in 93% [1]$H_2O$/7% [2]$H_2O$ (v/v) containing 20 mM sodium phosphate buffer at pH 5.0/0.01 % $NaN_3$.

*4.2 NMR spectroscopy*

4.2.1 General

All NMR data were acquired at 298 K on a Varian Unity INOVA 500 (UIPS) or INOVA600 (GBB) NMR spectrometer. The NMR data were processed using the NMRPipe package (Delaglio et al., 1995) and analyzed with the Sparky NMR assignment and integration software (T. D. Goddard and D. G. Kneller, SPARKY 3, University of California, San Francisco).

4.2.2 Spectral assignments

Backbone $^1$H, $^{15}$N, [13]$C^{\alpha/\beta}$ and [1]$H^{\alpha/\beta}$ nuclei were assigned using standard triple resonance experiments (see Cavanagh et al., 1996). Assignment of the aliphatic side-chain was accomplished by interpretation of a HC(C)H-TOCSY (Kay et al., 1993) experiments using the previous assignments of the [13]$C^{\alpha/\beta}$ and [1]$H^{\alpha/\beta}$ nuclei as a starting point. The aromatic side-chains were assigned using 2D experiments, which correlate the [13]$C^{\beta}$/[1]$H^{\beta}$ resonances with the protons of the aromatic moiety (Yamazaki et al., 1993), plus a 3D HC(C)H-TOCSY with the $^{13}$C carrier placed in the aromatic region in order to connect the aromatic spin-systems. Coherence mixing in the HC(C)H-TOCSY experiments was accomplished by the use of a 15.5 ms DIPSI-3 mixing sequence (Shaka et al., 1988).

*4.2.3 NMR titration experiment*

A titration experiment was performed in which the CHIPS31-121 $^1$H and $^{15}$N chemical shifts were monitored as function of an increasing amount of $SO_4$-C5AR pep7-28. Series of $^1$H-$^{15}$N HSQC experiments were acquired of samples in which the [$SO_4$-C5AR pep7-28] : [CHIPS31-121] ratio was varied between 0:1 and 1:1. This was accomplished by adding to a solution of 400 ml of 0.45 mM CHIPS31-121 9 ml of 4 mM $SO_4$-C5AR pep7-28 (this gives a starting [$SO_4$-C5AR pep7-28] : [CHIPS31-121] ratio of 1:5) and subsequent aliquots of 9 ml.

***RESULTS***

**Table 2** shows all amino acids of CHIPS31-121 that are or might be involved in the binding of CHIPS31-121 to the C5aR, as obtained by titration of $SO_4$-C5AR pep7-28 to $^{15}$N-labeled CHIPS31-121 in a $^{15}$N-1H-HSQC NMR experiment. Residues R46, K50, K51, K54, E60, K100, K101, G102, K105, Y108, V109 and Y121 are proven to be involved in the binding.

**EXAMPLE 5**

Molecular biological confirmation of amino acids of CHIPS31-121 involved in the binding of CHIPS to the C5aR

***MATERIALS AND METHODS***

5.1 *Expression of CHIPS31-121 and CHIPS31-121 mutants in a periplasmic leaky expression system*

For expression of CHIPS31-121 and CHIPS31-121 mutants in a periplasmic leaky expression system, CHIPS31-121 was cloned into the pET-22b(+)-vector (Novagen). Therefore, CHIPS31-121 was amplified by PCR as described in section 1.2 CHIPS production, using the primers:

> 5' CGATGGCCAATAGTGGTCTTCCTACAACG

and

> 3' CTACTAGCT<u>GAATTC</u>TTAGTATGCATATTCATTAG.

All CHIPS31-121 mutants were constructed using site-directed mutagenesis on CHIPS31-121 by overlap extension PCR (Ho SN et al., 1989, Gene 77;51). The purified PCR products were digested with MscI and EcoRI, and subsequently ligated into a MscI- and EcoRI-digested pET-22b(+)-vector. Plasmids were propagated in TOP10F' *E. coli.*
For expression, BL21(DE3) *E. coli* were transformed with the constructs. Expression of CHIPS31-121 and CHIPS31-121 mutants was accomplished by induction with 1 mM isopropyl β-D-thiogalactopyranoside (IPTG) overnight at 30°C. HSA (0.1 %) was added against loss of protein. Subsequently, the supernatant was collected and PMSF was added to avoid protease activity against the expressed proteins.
The concentration of expressed CHIPS31-121 or CHIPS31-121 mutants in the bacterial supernatant was determined using a capture ELISA, in which polyclonal rabbit anti-CHIPS antibodies were coated to the ELISA plates.
After binding, CHIPS31-121 or CHIPS31-121 mutants are detected with biotinylated polyclonal rabbit anti-CHIPS antibodies, and subsequent streptavidin-peroxidase binding.

5.2 *Competition of CHIPS31-121 or CHIPS31-121 mutants and anti-C5aR mAb binding to U937 cells, expressing the C5aR*

U937 cells, expressing the C5aR, were incubated with increasing concentrations of CHIPS31-121 or CHIPS31-121 mutants (present in the bacterial supernatants) for 15 min on ice in RPMI, containing 0.05% human serum albumin (RPMI/0.05% HSA). Then, the samples were incubated with 10 µg/ml FITC-labeled anti-C5aR S5/1 mAb (anti-CD88 (Serotec, Oxford, UK)) on ice for 30 min. After washing, the cells were analyzed on a FACscan flow cytometer (Becton Dickinson, San Jose, CA, USA).

***RESULTS***

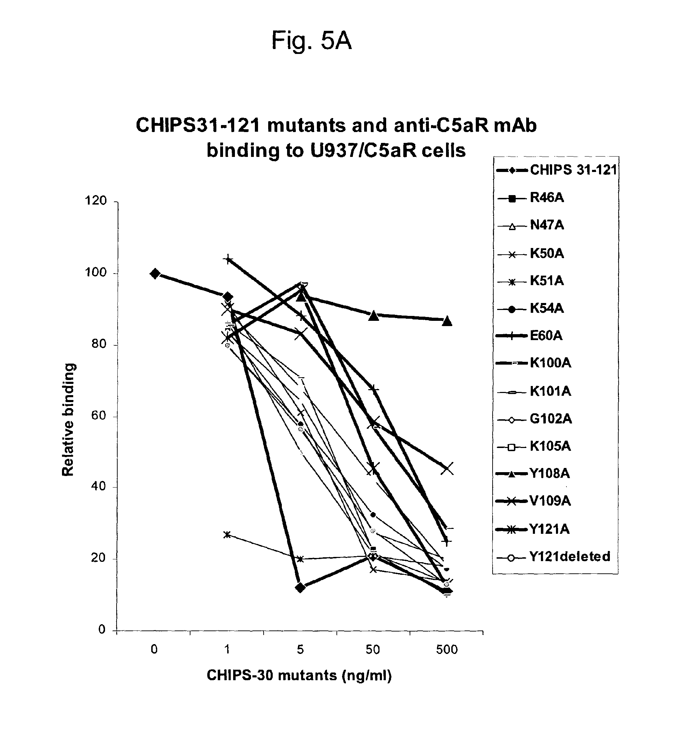

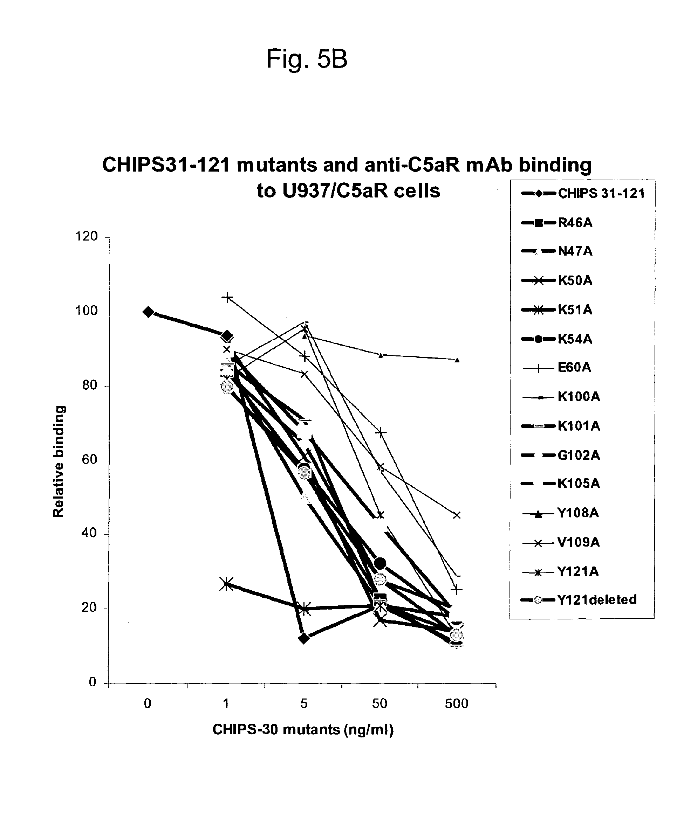

**Figure 5A+B** show the inhibition of the binding of anti-C5aR monoclonal antibody to the C5aR, expressed on U937 cells, by CHIPS31-121 and indicated mutants of CHIPS31-121. Thick lines in **Figure 5A** indicate CHIPS31-121 and the CHIPS31-121 mutants that are affected the most in binding to C5aR (being E60, K100, Y108, V109 and Y121). Thick lines in **Figure 5B** indicate CHIPS31-121 and the CHIPS31-121 mutants that are affected the most in binding to C5aR (being R46, N47, K50, K51, K54, K101, G102, K105 and Y121deleted) All mutations were single amino acid substitutions into alanines (unless otherwise stated).
**Table 3** shows the $IC_{50}$ values of diverse CHIPS31-121 mutants, in which single amino acids were substituted into alanines (unless otherwise stated). $IC_{50}$ determinations were done using the experiments shown in **figure 5A+B.**

## Table 1:

**Amino acids of the C5aR** that are or might be involved in the binding of CHIPS to the C5aR, obtained by expression of the C5aR-N-terminus and the whole C5aR +/- mutants

| Amino acid position | 1 | 2 | 3 | 4 | 5 | 6 | 7 | 8 | 9 | 10 |
|---|---|---|---|---|---|---|---|---|---|---|
| 3-letter/1-letter code | Met/M | Asn/N | Ser/S | Phe/F | Asn/N | Tyr/Y | Thr/T | Thr/T | Pro/P | Asp/D |
| Involved in CHIPS binding | - | - | - | - | - | - | - | - | - | ++ |

| Amino acid position | 11 | 12 | 13 | 14 | 15 | 16 | 17 | 18 | 19 | 20 |
|---|---|---|---|---|---|---|---|---|---|---|
| 3-letter/1-letter code | Tyr/Y | Gly/G | His/H | Tyr/Y | Asp/D | Asp/D | Lys/K | Asp/D | Thr/T | Leu/L |
| Involved in CHIPS binding | - | ++ | - | + | ++ | - | - | ++ | - | - |

| Amino acid position | 21 | 22 | 23 | 24 | 25 | 26 | 27 | 28 | 29 | 30 |
|---|---|---|---|---|---|---|---|---|---|---|
| 3-letter/1-letter code | Asp/D | Leu/L | Asn/N | Thr/T | Pro/P | Val/V | Asp/D | Lys/K | Thr/T | Ser/S |
| Involved in CHIPS binding | - | - | - | - | - | - | - | - | - | - |

| Amino acid position | 31 | 32 | 33 | 34 | 35 | 36 | 37 |
|---|---|---|---|---|---|---|---|
| 3-letter/1-letter code | Asn/N | Thr/T | Leu/L | Arg/R | Val/V | Pro/P | Asp/D |
| Involved in CHIPS binding | - | - | - | - | - | - | - |

# Amino acids 38 to 350:

ILALVIFAVVFLVGVLGNALVVWVTAFEAKRTINAIWFLNLAVADFLSCLALPILFTSIV
QHHHWPFGGAACSILPSLILLNMYASILLLATISADRFLLVFKPIWCQNFRGAGLAWIAC
AVAWGLALLLTIPSFLYRVVREEYFPPKVLCGVDYSHDKRRERAVAIVRLVLGFLWPLLT
LTICYTFILLRTWSRRATRSTKTLKVVVAVVASFFIFWLPYQVTGIMMSFLEPSSPTFLL
LNKLDSLCVSFAYINCCINPIIYVVAGQGFQGRLRKSLPSLLRNVLTEESVVRESKSFTR
STVDTMAQKTQAV

# are not involved in the binding of CHIPS

## Table 2:

**Amino acids of CHIPS** that are or might be involved in the binding of CHIPS to the C5aR, obtained by titration of C5aR-pep7-28 to 15N-labeled CHIPS-delta-30 in a 15N-1H-HSQC NMR experiment.

| Amino acid position | 31 | 32 | 33 | 34 | 35 | 36 | 37 | 38 | 39 | 40 |
|---|---|---|---|---|---|---|---|---|---|---|
| 3-letter/1-letter code | Asn/N | Ser/S | Gly/G | Leu/L | Pro/P | Thr/T | Thr/T | Leu/L | Gly/G | Lys/K |
| Involved in CHIPS binding | nd | nd | - | - | nd | - | + | +/- | - | - |
| Confirmed by Mol. Biol. * | | | | | | | | | | |

| Amino acid position | 41 | 42 | 43 | 44 | 45 | 46 | 47 | 48 | 49 | 50 |
|---|---|---|---|---|---|---|---|---|---|---|
| 3-letter/1-letter code | Leu/L | Asp/D | Glu/E | Arg/R | Leu/L | Arg/R | Asn/N | Tyr/Y | Leu/L | Lys/K |
| Involved in CHIPS binding | - | - | - | + | + | ++ | + | +/- | +/- | ++ |
| Confirmed by Mol. Biol. | | | | | | ++ | + | | | ++ |

| Amino acid position | 51 | 52 | 53 | 54 | 55 | 56 | 57 | 58 | 59 | 60 |
|---|---|---|---|---|---|---|---|---|---|---|
| 3-letter/1-letter code | Lys/K | Gly/G | Thr/T | Lys/K | Asn/N | Ser/S | Ala/A | Gln/Q | Phe/F | Glu/E |
| Involved in CHIPS binding | ++ | ++ | + | ++ | - | - | +/- | - | + | ++ |
| Confirmed by Mol. Biol. | ++ | | | ++ | | | | | | ++ |

| Amino acid position | 61 | 62 | 63 | 64 | 65 | 66 | 67 | 68 | 69 | 70 |
|---|---|---|---|---|---|---|---|---|---|---|
| 3-letter/1-letter code | Lys/K | Met/M | Val/V | Ile/I | Leu/L | Thr/T | Glu/E | Asn/N | Lys/K | Gly/G |
| Involved in CHIPS binding | + | - | + | +/- | +/- | - | - | - | - | - |
| Confirmed by Mol. Biol. | | | | | | | | | | |

| Amino acid position | 71 | 72 | 73 | 74 | 75 | 76 | 77 | 78 | 79 | 80 |
|---|---|---|---|---|---|---|---|---|---|---|
| 3-letter/1-letter code | Tyr/Y | Tyr/Y | Thr/T | Val/V | Tyr/Y | Leu/L | Asn/N | Thr/T | Pro/P | Leu/L |
| Involved in CHIPS binding | + | - | +/- | - | - | + | - | - | nd | +/- |
| Confirmed by Mol. Biol. | | | | | | | | | | |

| Amino acid position | 81 | 82 | 83 | 84 | 85 | 86 | 87 | 88 | 89 | 90 |
|---|---|---|---|---|---|---|---|---|---|---|
| 3-letter/1-letter code | Ala/A | Glu/E | Asp/D | Arg/R | Lys/K | Asn/N | Val/V | Glu/E | Leu/L | Leu/L |
| Involved in CHIPS binding | - | - | +/- | +/- | +/- | - | - | + | +/- | - |
| Confirmed by Mol. Biol. | | | | | | | | | | |

| Amino acid position | 91 | 92 | 93 | 94 | 95 | 96 | 97 | 98 | 99 | 100 |
|---|---|---|---|---|---|---|---|---|---|---|
| 3-letter/1-letter code | Gly/G | Lys/K | Met/M | Tyr/Y | Lys/K | Thr/T | Tyr/Y | Phe/F | Phe/F | Lys/K |
| Involved in CHIPS binding | +/- | nd | +/- | - | - | +/- | +/- | - | - | ++ |
| Confirmed by Mol. Biol. | | | | | | | | | | ++ |

| Amino acid position | 101 | 102 | 103 | 104 | 105 | 106 | 107 | 108 | 109 | 110 |
|---|---|---|---|---|---|---|---|---|---|---|
| 3-letter/1-letter code | Lys/K | Gly/G | Glu/E | Ser/S | Lys/K | Ser/S | Ser/S | Tyr/Y | Val/V | Ile/I |
| Involved in CHIPS binding | ++ | ++ | + | - | ++ | - | - | ++ | ++ | +/- |
| Confirmed by Mol. Biol. | ++ | ++ | | | ++ | | | ++ | ++ | |

| Amino acid position | 111 | 112 | 113 | 114 | 115 | 116 | 117 | 118 | 119 | 120 |
|---|---|---|---|---|---|---|---|---|---|---|
| 3-letter/1-letter code | Asn/N | Gly/G | Pro/P | Gly/G | Lys/K | Thr/T | Asn/N | Glu/E | Tyr/Y | Ala/A |
| Involved in CHIPS binding | + | - | nd | - | + | - | - | - | - | - |
| Confirmed by Mol. Biol. | | | | | | | | | | |

| Amino acid position | 121 |
|---|---|
| 3-letter/1-letter code | Tyr/Y |
| Involved in CHIPS binding | ++ |
| Confirmed by Mol. Biol. | ++ |

| | |
|---|---|
| ++ | involved in the binding of CHIPS to the C5aR |
| + | most probably involved in the binding of CHIPS to the C5aR |
| +/- | probably involved in the binding of CHIPS to the C5aR |
| - | not involved in the binding of CHIPS to the C5aR |
| nd | not determined |
| * Confirmed by Mol. Biol.: Confirmed by Molecular Biology | |

Table 3

| IC50 values of CHIPS31-121 mutants (substitutions into alanine, unless otherwise stated), as tested in the inhibition of anti-C5aR mAb binding to U937 cells, expressing the C5aR. | |
| --- | --- |
| | IC50 (ng/ml) |
| CHIPS31-121 | 2 |
| N47 | 5 |
| R46 | 10 |
| Y121 deleted | 10 |
| K54 | 10 |
| K50 | 10 |
| G102 | 12 |
| K101 | 15 |
| K105 | 25 |
| Y121 | 50 |
| K100 | 100 |
| E60 | 150 |
| V109 | 250 |
| Y108 | >500 |

**Table 4**

Diseases caused by inflammatory reactions, involving complement activation and/or neutrophil and or monocyte involvement.

- acute reactive arthritis
- acute transplant rejection
- adult respiratory distress syndrome (ARDS)
- alcoholic hepatitis
- allotransplantation
- Alzheimer's disease
- arteriosclerosis
- arthus reaction
- asthma
- atherosclerosis
- atopic dermatitis
- bacterial meningitis
- bronchogenic carcinoma
- bullos pemphigoid
- burns
- cardiopulmonary bypass
- cardiovascular diseases
- chronic bronchitis
- chronic lymph leukemia
- chronic obstructive pulmonary disease (COPD)
- contact dermatitis
- Crohn's disease
- cutaneous T-cell lymphoma
- cystic fibrosis
- dermatoses
- diseases of the central nervous system
- endometriosis
- experimental allergic encephalomyelitis (EAE)
- experimental allergic neuritis (EAN)
- frost bite
- gastric carcinoma
- gastrointestinal diseases
- genitourinary diseases
- gout
- Heliobacter pylori gastritis
- hemodialysis
- hereditary angioedema
- hypersensitivity pneumonia

- idiopathic pulmonary fibrosis
- immune complex (IC)-induced vasculitis
- ischaemic shock
- ischaemia-reperfusion episodes
- ischemia-reperfusion injuries
- joint diseases
- (large) vessel surgery
- metal fume fever
- multiple sclerosis
- multiple system organ failure
- myasthenia gravis
- myocardial infarction
- pancreatitis
- peritonitis
- pleural emphesema
- post- cardiopulmonary bypass (CBP) inflammation
- psoriasis
- repetitive strain injury (RSI)
- respiratory diseases
- rheumatoid arthritis
- sepsis
- septic shock
- sinusitis
- skin diseases
- stroke
- systemic lupus erythematosus (SLE)
- transplantation
- (traumatic) brain injury
- ulcerative colitis
- urinary tract infection
- vascular leak syndrome
- vasculitis
- xenotransplantation

**Claims**

1. Compounds that are able to prevent intramolecular contact of the N-terminal residues 10 to 18 of human C5aR with the extracellular loops thereof.

2. Compounds as claimed in claim 1, **characterized in that** they are able to bind the aspartates in positions 10, 15 and 18 and the glycine in position 12 of the human C5aR.

3. Compounds as claimed in any one of the claims 1-3, wherein the compounds are of the general formula:

$$X_n\text{-}E\text{-}X_{39}\text{-}K\text{-}X_7\text{-}Y\text{-}V\text{-}X_{11}\text{-}Y\text{-}X_m \qquad\qquad (I)$$

wherein:

$X_n$ may or may not be present and is a stretch of proteinogenic amino acids, non-proteinogenic amino acids, D-amino acids, peptidomimetic building blocks or combinations thereof;
K is lysine or a non-proteinogenic analog thereof;
E is glutamic acid or a non-proteinogenic analog thereof;
Y is tyrosine or a non-proteinogenic analog thereof; and
V is valine or a non-proteinogenic analog thereof
$X_{39}$, $X_7$, and $X_{11}$ are each a stretch of 39, 7 or 11, respectively, proteinogenic amino acids, non-proteinogenic amino acids, D-amino acids, peptidomimetic building blocks or combinations thereof;
$X_m$ may or may not be present and is a stretch of proteinogenic amino acids, non-proteinogenic amino acids, D-amino acids, peptidomimetic building blocks or combinations thereof;

and have the same three-dimensional conformation as naturally occurring CHIPS.

4. Compounds as claimed in claim 3, wherein $X_n$ and $X_m$ in formula I may each contain 0-100, preferably 1-90, more preferably -75, most preferably 1-59 residues.

5. Compounds as claimed in any one of the claims 1-3, wherein the compounds are of the general formula:

$$X_n\text{-}R\text{-}X_3\text{-}K\text{-}K\text{-}G\text{-}X\text{-}K\text{-}X_5\text{-}E\text{-}X_{39}\text{-}K\text{-}K\text{-}G\text{-}X_2\text{-}K\text{-}X_2\text{-}Y\text{-}V\text{-}X_{11}\text{-}Y\text{-}X_m \qquad\qquad (II)$$

wherein:

$X_n$ may or may not be present and is a stretch of proteinogenic amino acids, non-proteinogenic amino acids, D-amino acids, peptidomimetic building blocks or combinations thereof;
R is arginine or a non-proteinogenic analog thereof;
K is lysine or a non-proteinogenic analog thereof;
E is glutamic acid or a non-proteinogenic analog thereof;
X, $X_2$, $X_3$, $X_5$, $X_{39}$ and $X_{11}$ are each a stretch of 1, 2, 3, 5, 39 and 11, respectively, proteinogenic amino acids, non-proteinogenic amino acids, D-amino acids, peptidomimetic building blocks or combinations thereof;
Y is tyrosine or a non-proteinogenic analog thereof; and
V is valine or a non-proteinogenic analog thereof
$X_m$ may or may not be present and is a stretch of proteinogenic amino acids, non-proteinogenic amino acids, D-amino acids, peptidomimetic building blocks or combinations thereof;

and have the same three-dimensional conformation as naturally occurring CHIPS.

6. Compounds as claimed in claim 5, wherein $X_n$ and $X_m$ in formula II may each contain 0-100, preferably 1-50, more preferably 1-25, most preferably 1-15.

7. Compounds as claimed in any one of the claims 1-3, wherein the compounds are of the general formula:

$$X_n\text{-}T\text{-}X_6\text{-}R\text{-}L\text{-}R\text{-}N\text{-}X_2\text{-}K\text{-}K\text{-}G\text{-}T\text{-}K\text{-}X_4\text{-}F\text{-}E\text{-}K\text{-}X\text{-}V\text{-}X_7\text{-}Y\text{-}X_4\text{-}L\text{-}X_{11}\text{-}E\text{-}X_{11}\text{-}K\text{-}K\text{-}$$

$$G\text{-}E\text{-}X\text{-}K\text{-}X_2\text{-}Y\text{-}V\text{-}X\text{-}N\text{-}X_3\text{-}K\text{-}X_5\text{-}Y\text{-}X_m \qquad \text{(III)}$$

wherein

$X_n$ may or may not be present and is a stretch of proteinogenic amino acids, non-proteinogenic amino acids, D-amino acids, peptidomimetic building blocks or combinations thereof;

T is threonine or a non-proteinogenic analog thereof;

R is arginine or a non-proteinogenic analog thereof;

L is leucine or a non-proteinogenic analog thereof;

N is asparagine or a non-proteinogenic analog thereof;

$X$, $X_2$, $X_3$, $X_4$, $X_5$, $X_6$, $X_7$ and $X_{11}$ are each a stretch of 1, 2, 3, 4, 5, 6, 7 or 11, respectively proteinogenic amino acids, non-proteinogenic amino acids, D-amino acids, peptidomimetic building blocks or combinations thereof;

K is lysine or a non-proteinogenic analog thereof;

G is glycine or a non-proteinogenic analog thereof;

F is phenylalanine or a non-proteinogenic analog thereof;

E is glutamic acid or a non-proteinogenic analog thereof;

Y is tyrosine or a non-proteinogenic analog thereof; and

V is valine or a non-proteinogenic analog thereof

and have the same three-dimensional conformation as naturally occurring CHIPS.

8. Compounds as claimed in any one of the claims 1-4, wherein the compounds are of the general formula:

$$X_n\text{-}T\text{-}L\text{-}X_5\text{-}R\text{-}L\text{-}R\text{-}N\text{-}Y\text{-}L\text{-}K\text{-}K\text{-}G\text{-}T\text{-}K\text{-}X_2\text{-}A\text{-}X\text{-}F\text{-}E\text{-}K\text{-}X\text{-}V\text{-}I\text{-}L\text{-}X_5\text{-}Y\text{-}X\text{-}T\text{-}$$

$$X_2\text{-}L\text{-}X_3\text{-}L\text{-}X_2\text{-}D\text{-}R\text{-}K\text{-}X_2\text{-}E\text{-}L\text{-}X\text{-}G\text{-}X\text{-}M\text{-}X_2\text{-}T\text{-}Y\text{-}X_2\text{-}K\text{-}K\text{-}G\text{-}E\text{-}X\text{-}K\text{-}X_2\text{-}Y\text{-}V\text{-}$$

$$I\text{-}N\text{-}X_3\text{-}K\text{-}X_5\text{-}Y\text{-}X_m \qquad \text{(IV)}$$

wherein

$X_n$ may or may not be present and is a stretch of proteinogenic amino acids, non-proteinogenic amino acids, D-amino acids, peptidomimetic building blocks or combinations thereof;

T is threonine or a non-proteinogenic analog thereof;

R is arginine or a non-proteinogenic analog thereof;

L is leucine or a non-proteinogenic analog thereof;

I is isoleucine or a non-proteinogenic analog thereof;

M is methionine or a non-proteinogenic analog thereof;

A is alanine or a non-proteinogenic analog thereof;

D is asparagine or a non-proteinogenic analog thereof;

N is asparagine or a non-proteinogenic analog thereof;

K is lysine or a non-proteinogenic analog thereof;

G is glycine or a non-proteinogenic analog thereof;

F is phenylalanine or a non-proteinogenic analog thereof;

E is glutamic acid or a non-proteinogenic analog thereof;

Y is tyrosine or a non-proteinogenic analog thereof; and

V is valine or a non-proteinogenic analog thereof

$X$, $X_2$, $X_3$ and $X_5$, are each a stretch of 1, 2, 3 or 5, respectively proteinogenic amino acids, non-proteinogenic amino acids, D-amino acids, peptidomimetic building blocks or combinations thereof;

and have the same three-dimensional conformation as naturally occurring CHIPS.

9. Compounds as claimed in claim 7 or 8, wherein $X_n$ and $X_m$ in formulas III and IV may each contain 0-100, preferably 1-50, more preferably 1-25, most preferably 1-6.

10. Compound as claimed in any one of the claims 1-9, which compound is selected from CHIPSΔ1, CHIPSΔ17, CHIPSΔ30, which lack 1, 17 and 30 amino acids, respectively, of the N-terminal domain of naturally occurring CHIPS.

**11.** Compounds as claimed in any one of the claims 1-10, wherein the non-proteinogenic analog is selected from the group consisting of 2-naphtylalanine (Nal(2)), β-cyclohexylalanine (Cha), p-amino-phenylalanine ((Phe(p-NH2), p-benzoyl-phenylalanine (Bpa), ornithine (Orn), norleucine (Nle), 4-fluoro-phenylalanine (Phe(p-F)), 4-chloro-phenylalanine (Phe(p-Cl)), 4-bromo-phenylalanine (Phe(p-Br)), 4-iodo-phenylalanine (Phe(p-I)), 4-methyl-phenylalanine (Phe(p-Me)), 4-methoxy-phenylalanine (Tyr(Me)), 4-nitro-phenylalanine (Phe(p-NO2)).

**12.** Compounds as claimed in any one of the claims 1-11, wherein the non-proteinogenic analog is a D-amino acid selected from the group consisting of D-phenylalanine, D-alanine, D-arginine, D-asparagine, D-aspartic acid, D-cysteine, D-glutamic acid, D-glutamine, D-histidine, D-isoleucine, D-leucine, D-lysine, D-methionine, D-proline, D-serine, D-threonine, D-tryptophan, D-tyrosine, D-valine, D-2-naphtylalanine (D-Nal(2)), β-cyclohexyl-D-alanine (D-Cha), 4-amino-D-phenylalanine (D-Phe(p-NH2)), p-benzoyl-D-phenylalanine (D-Bpa), D-Ornithine (D-Orn), D-Norleucine (D-Nle), 4-fluoro-D-phenylalanine (D-Phe(p-F)), 4-chloro-D-phenylalanine (D-Phe(p-Cl)), 4-bromo-D-phenylalanine (D-Phe(p-Br)), 4-iodo-D-phenylalanine (D-Phe(p-I)), 4-methyl-D-phenylalanine (D-Phe(p-Me)), 4-methoxy-D-phenylalanine (D-Tyr(Me)), 4-nitro-D-phenylalanine (D-Phe(p-NO2)).

**13.** Compounds as claimed in any one of the claims 1-12, wherein the non-proteinogenic analog is a peptidomimetic building block selected from the group consisting of oligo-β-peptides, oligosulfonamides, vinylogous sulfonamides, hydrazinepeptide/azatides, oligocarbamates, ureapeptoids, oligourea, phosphodiesters, peptoids, oligosulfones, peptoid sulfonamides, vinylogous peptides.

**14.** Compounds as claimed in claim 13, wherein the peptidomimetic building block is selected from the group consisting of N-substituted glycines, such as N-benzylglycine (NPhe), N-methylglycine (NAla), N-(3-guanidinopropyl)glycine (NArg), N-(Carboxymethyl)glycine (NAsp), N-(carbamylmethyl) glycine (NAsn), N-(thioethyl)-glycine (NhCys), N-(2-carboxyethyl)glycine (NGlu), N-(2-carbamylethyl)glycine (NGln), N-(imidazolylethyl)glycine (NhHis), N-(1-methylpropyl) glycine (NIle), N-(2-methylpropyl)glycine (NLeu), N-(4-aminobutyl)glycine (NLys), N-(2-methylthioethyl)glycine (NMet), N-(hydroxyethyl)glycine (NhSer), N-(2-hydroxypropyl) glycine (NhThr), N-(3-indolylmethyl)glycine (Ntrp), N-(p-hydroxyphenmethyl)-glycine (NTyr), N-(1-methylethyl) glycine (NVal).

**15.** Compounds as claimed in any one of the claims 1-14 in cyclic form.

**16.** Compounds as claimed in claims 1 and 2, which compounds are antibodies or derivatives thereof against C5aR or a C5aR related compound that comprises the motif $X_q$-D-X-G-$X_2$-D-$X_2$-D-$X_r$, wherein $X_q$, X, $X_2$ and $X_r$ are stretches of 0-9, 1, 2 and 0-20.

**17.** Compounds as claimed in any one of the claims 1-16 for use in prophylaxis or therapy.

**18.** Compounds as claimed in any one of the claims 1-16 for use in the prophylaxis or treatment of indications involving the C5a-receptor (C5aR) on neutrophils, monocytes and endothelial cells.

**19.** Compounds as claimed in claim 18, wherein the indication involves acute or chronic inflammation reactions.

**20.** Compounds as claimed in claim 19, wherein the indication is selected from the group listed in Table 4.

**21.** Compounds as claimed in any one of the claims 1-16 for use in the prophylaxis or treatment of indications involving C5aR on cells other than neutrophils, monocytes and endothelial cells.

**22.** Compounds as claimed in claim 21, wherein the other cells are lymphocytes, dendritic cells, eosinophils, basophils, macrophages, microglia cells, astrocytes, Kupfer cells, hepatocytes and epithelial cells.

**23.** Compounds as claimed in any one of the claims 1-16 for use in prophylactic or therapeutic vaccines for infections caused by CHIPS-producing bacteria.

**24.** Compounds as claimed in claim 23, wherein the CHIPS-producing bacterium is *Staphylococcus aureus.*

**25.** Use of the compounds as claimed in claims 1-16 for the manufacture of a therapeutic preparation for prophylaxis or therapy.

26. Use as claimed in claims 25, wherein the therapeutic preparation is for prophylaxis and treatment of indications involving C5aR on neutrophils, monocytes and endothelial cells.

27. Use as claimed in claim 25, wherein the indication involves acute or chronic inflammation reactions.

28. Use as claimed in claim 27 wherein the indication is selected from the group listed in Table 4.

29. Use as claimed in claim 25, wherein the therapeutic composition is for prophylaxis or treatment of indications involving C5aR on cells other than neutrophils, monocytes and endothelial cells.

30. Use as claimed in claim 29, wherein the other cells are lymphocytes, dendritic cells, eosinophils, basophils, macrophages, microglia cells, astrocytes, Kupfer cells, hepatocytes and epithelial cells.

31. Use as claimed in claim 25, wherein the therapeutic preparation is a prophylactic or therapeutic vaccine that can be used in the prophylaxis or treatment of infections caused by CHIPS-producing bacteria.

32. Use as claimed in claim 31, wherein the CHIPS-producing bacterium is *Staphylococcus aureus.*

33. A therapeutic composition comprising a suitable excipient and one or more compounds as claimed in claims 1-16.

34. A prophylactic composition comprising a suitable excipient and one or more compounds as claimed in claims 1-16.

35. Use of a compound as claimed in any one of the claims 1-16 for the preparation of a coating composition for use on surfaces of medical devices that are introduced into the human body through the skin, or placed in the body during surgical procedures.

36. Use as claimed in claim 35, wherein the surface is the surface of a catheter tip.

37. Use as claimed in claims 35 and 36, wherein the composition is a slow-release composition.

38. Therapeutic or prophylactic composition comprising a suitable excipient and one or more compounds as claimed in any one of the claims 1-16.

39. Method for prophylaxis or treatment of a subject suffering from indications involving C5aR on neutrophils, monocytes and endothelial cells comprising administering a prophylactically or therapeutically effective amount of one or more of the compounds as claimed in any one of the claims 1-16.

40. Method as claimed in claim 39, wherein the indication involves acute or chronic inflammation reactions.

41. Method as claimed in claim 39 wherein the indication is selected from the group listed in Table 4.

42. Method for prophylaxis or treating a subject suffering from indications involving C5aR on cells other than neutrophils, monocytes and endothelial cells comprising administering a prophylactically or therapeutically effective amount of one or more of the compounds as claimed in any one of the claims 1-16.

43. Method as claimed in claim 42, wherein the other cells are lymphocytes, dendritic cells, eosinophils, basophils, macrophages, microglia cells, astrocytes, Kupfer cells, hepatocytes and epithelial cells.

44. Method for the prophylactic or therapeutic treatment of a subject against infections with CHIPS-producing bacteria comprising the administration of a prophylactically or therapeutically effective amount of one or more compounds as claimed in any one of the claims 1-16.

45. Method for the identification of compounds that can bind to the C5aR, comprising

a) provision of a library of candidate compounds,
b) contacting the members of the library with a C5aR related compound that comprises the motif $X_q$-D-X-G-$X_2$-O-$X_2$-D-$X_r$, wherein $X_q$, X, $X_2$ and $X_r$ are stretches of 0-9, 1, 2 and 0-20, respectively, proteinogenic amino acids or non-proteinogenic analogs thereof, D is asparaginic acid and G is glycine, in a conformation that is the same or similar as the conformation found in naturally occurring C5aR, and
c) identifying the members of the library that bind to the C5aR related compound.

46. Method as claimed in claim 45 further comprising the step of providing the thus identified members for use in prophylaxis or therapy.

47. Compounds as identified in the method according to claim 45.

48. Use of compounds as claimed in claim 47 for the preparation of a pharmaceutical composition for treatment and prophylaxis of indications involving the C5aR.

# Fig. 1

# Fig. 2A

# Fig. 2B

# Fig. 3

# Fig. 4

# Fig. 5A

**CHIPS31-121 mutants and anti-C5aR mAb binding to U937/C5aR cells**

Legend:
- CHIPS 31-121
- R46A
- N47A
- K50A
- K51A
- K54A
- E60A
- K100A
- K101A
- G102A
- K105A
- Y108A
- V109A
- Y121A
- Y121deleted

Y-axis: Relative binding

X-axis: CHIPS-30 mutants (ng/ml)

# Fig. 5B

CHIPS31-121 mutants and anti-C5aR mAb binding to U937/C5aR cells

SEQUENCE LISTING

<110>    Alligator Bioscience AB
<120>    Compounds that block the C5a receptor and their use in therapy

<130>    ALLA/P31503EP

<140>    EP 04076120.7
<141>    16 April 2004

<160>    14

<170>    SeqWin99

<210>    1
<211>    10
<212>    PRT
<213>    Artificial Sequence

<220>
<223>    Compound formula I

<220>
<221>    MISC_FEATURE
<222>    (1)..(1)
<223>    May or may not be present
         A stretch of proteinogenic amino-acids, non-proteinogenic amino acids,
         D-amino acids, peptidomimetic building blocks or combinations thereof

<220>
<221>    MISC_FEATURE
<222>    (3)..(3)
<223>    A stretch of 39 proteinogenic amino-acids, non-proteinogenic amino
         acids, D-amino acids, peptidomimetic building blocks or combinations
         thereof

<220>
<221>    MISC_FEATURE
<222>    (5)..(5)
<223>    A stretch of 7 proteinogenic amino-acids, non-proteinogenic amino
         acids, D-amino acids, peptidomimetic building blocks or combinations
         thereof

<220>
<221>    MISC_FEATURE
<222>    (8)..(8)
<223>    A stretch of 11 proteinogenic amino-acids, non-proteinogenic amino
         acids, D-amino acids, peptidomimetic building blocks or combinations
         thereof

<220>
<221>    MISC_FEATURE
<222>    (10)..(10)
<223>    May or may not be present
         A stretch of proteinogenic amino-acids, non-proteinogenic amino acids,
         D-amino acids, peptidomimetic building blocks or combinations thereof

<400>    1
Xaa Glu Xaa Lys Xaa Tyr Val Xaa Tyr Xaa
1               5                   10

```
<210>    2
<211>    22
<212>    PRT
<213>    Artificial Sequence


<220>
<223>    Compound formula II


<220>
<221>    MISC_FEATURE
<222>    (1)..(1)
<223>    May or may not be present
         A stretch of proteinogenic amino-acids, non-proteinogenic amino acids,
         D-amino acids, peptidomimetic building blocks or combinations thereof


<220>
<221>    MISC_FEATURE
<222>    (3)..(3)
<223>    A stretch of 3 proteinogenic amino-acids, non-proteinogenic amino
         acids, D-amino acids, peptidomimetic building blocks or combinations
         thereof


<220>
<221>    MISC_FEATURE
<222>    (7)..(7)
<223>    One proteinogenic amino-acid, non-proteinogenic amino acid, D-amino
         acid or peptidomimetic building block


<220>
<221>    MISC_FEATURE
<222>    (9)..(9)
<223>    A stretch of 5 proteinogenic amino-acids, non-proteinogenic amino
         acids, D-amino acids, peptidomimetic building blocks or combinations
         thereof


<220>
<221>    MISC_FEATURE
<222>    (11)..(11)
<223>    A stretch of 39 proteinogenic amino-acids, non-proteinogenic amino
         acids, D-amino acids, peptidomimetic building blocks or combinations
         thereof


<220>
<221>    MISC_FEATURE
<222>    (15)..(15)
<223>    A stretch of 2 proteinogenic amino-acids, non-proteinogenic amino
         acids, D-amino acids, peptidomimetic building blocks or combinations
         thereof


<220>
<221>    MISC_FEATURE
<222>    (17)..(17)
<223>    A stretch of 2 proteinogenic amino-acids, non-proteinogenic amino
         acids, D-amino acids, peptidomimetic building blocks or combinations
         thereof


<220>
<221>    MISC_FEATURE
<222>    (20)..(20)
```

```
<223>   A stretch of 11 proteinogenic amino-acids, non-proteinogenic amino
        acids, D-amino acids, peptidomimetic building blocks or combinations
        thereof

<220>
<221>   MISC_FEATURE
<222>   (22)..(22)
<223>   May or may not be present
        A stretch of proteinogenic amino-acids, non-proteinogenic amino acids,
        D-amino acids, peptidomimetic building blocks or combinations thereof

<400>   2
Xaa Arg Xaa Lys Lys Gly Xaa Lys Xaa Glu Xaa Lys Lys Gly Xaa Lys
1               5                   10                  15

Xaa Tyr Val Xaa Tyr Xaa
            20

<210>   3
<211>   42
<212>   PRT
<213>   Artificial Sequence

<220>
<223>   Compound formula III

<220>
<221>   MISC_FEATURE
<222>   (1)..(1)
<223>   May or may not be present
        A stretch of proteinogenic amino-acids, non-proteinogenic amino acids,
        D-amino acids, peptidomimetic building blocks or combinations thereof

<220>
<221>   MISC_FEATURE
<222>   (3)..(3)
<223>   A stretch of 6 proteinogenic amino-acids, non-proteinogenic amino
        acids, D-amino acids, peptidomimetic building blocks or combinations
        thereof

<220>
<221>   MISC_FEATURE
<222>   (8)..(8)
<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (14)..(14)
<223>   A stretch of 4 proteinogenic amino-acids, non-proteinogenic amino
        acids, D-amino acids, peptidomimetic building blocks or combinations
        thereof

<220>
<221>   MISC_FEATURE
<222>   (18)..(18)
<223>   One proteinogenic amino-acid, non-proteinogenic amino acid, D-amino
        acid or peptidomimetic building block

<220>
```

```
<221>   MISC_FEATURE
<222>   (20)..(20)
<223>   A stretch of 7 proteinogenic amino-acids, non-proteinogenic amino
        acids, D-amino acids, peptidomimetic building blocks or combinations
        thereof


<220>
<221>   MISC_FEATURE
<222>   (22)..(22)
<223>   A stretch of 4 proteinogenic amino-acids, non-proteinogenic amino
        acids, D-amino acids, peptidomimetic building blocks or combinations
        thereof


<220>
<221>   MISC_FEATURE
<222>   (24)..(24)
<223>   A stretch of 11 proteinogenic amino-acids, non-proteinogenic amino
        acids, D-amino acids, peptidomimetic building blocks or combinations
        thereof


<220>
<221>   MISC_FEATURE
<222>   (26)..(26)
<223>   A stretch of 11 proteinogenic amino-acids, non-proteinogenic amino
        acids, D-amino acids, peptidomimetic building blocks or combinations
        thereof


<220>
<221>   MISC_FEATURE
<222>   (31)..(31)
<223>   One proteinogenic amino-acid, non-proteinogenic amino acid, D-amino
        acid or peptidomimetic building block


<220>
<221>   MISC_FEATURE
<222>   (33)..(33)
<223>   A stretch of 2 proteinogenic amino-acids, non-proteinogenic amino
        acids, D-amino acids, peptidomimetic building blocks or combinations
        thereof


<220>
<221>   MISC_FEATURE
<222>   (36)..(36)
<223>   One proteinogenic amino-acid, non-proteinogenic amino acid, D-amino
        acid or peptidomimetic building block


<220>
<221>   MISC_FEATURE
<222>   (38)..(38)
<223>   A stretch of 3 proteinogenic amino-acids, non-proteinogenic amino
        acids, D-amino acids, peptidomimetic building blocks or combinations
        thereof


<220>
<221>   MISC_FEATURE
<222>   (40)..(40)
<223>   A stretch of 5 proteinogenic amino-acids, non-proteinogenic amino
        acids, D-amino acids, peptidomimetic building blocks or combinations
        thereof


<220>
```

```
<221>    MISC_FEATURE
<222>    (42)..(42)
<223>    May or may not be present
         A stretch of proteinogenic amino-acids, non-proteinogenic amino acids,
         D-amino acids, peptidomimetic building blocks or combinations thereof

<400>    3
Xaa Thr Xaa Arg Leu Arg Asn Xaa Lys Lys Gly Thr Lys Xaa Phe Glu
1               5                   10                  15

Lys Xaa Val Xaa Tyr Xaa Leu Xaa Glu Xaa Lys Lys Gly Glu Xaa Lys
        20                  25                  30

Xaa Tyr Val Xaa Asn Xaa Lys Xaa Tyr Xaa
        35                  40

<210>    4
<211>    64
<212>    PRT
<213>    Artificial Sequence

<220>
<223>    Compound formula IV

<220>
<221>    MISC_FEATURE
<222>    (1)..(1)
<223>    May or may not be present
         A stretch of proteinogenic amino-acids, non-proteinogenic amino acids,
         D-amino acids, peptidomimetic building blocks or combinations thereof

<220>
<221>    MISC_FEATURE
<222>    (4)..(4)
<223>    A stretch of 5 proteinogenic amino-acids, non-proteinogenic amino
         acids, D-amino acids, peptidomimetic building blocks or combinations
         thereof

<220>
<221>    MISC_FEATURE
<222>    (16)..(16)
<223>    A stretch of 2 proteinogenic amino-acids, non-proteinogenic amino
         acids, D-amino acids, peptidomimetic building blocks or combinations
         thereof

<220>
<221>    MISC_FEATURE
<222>    (18)..(18)
<223>    One proteinogenic amino-acid, non-proteinogenic amino acid, D-amino
         acid or peptidomimetic building block

<220>
<221>    MISC_FEATURE
<222>    (22)..(22)
<223>    One proteinogenic amino-acid, non-proteinogenic amino acid, D-amino
         acid or peptidomimetic building block

<220>
<221>    MISC_FEATURE
<222>    (26)..(26)
```

```
<223>   A stretch of 5 proteinogenic amino-acids, non-proteinogenic amino
        acids, D-amino acids, peptidomimetic building blocks or combinations
        thereof

<220>
<221>   MISC_FEATURE
<222>   (28)..(28)
<223>   One proteinogenic amino-acid, non-proteinogenic amino acid, D-amino
        acid or peptidomimetic building block

<220>
<221>   MISC_FEATURE
<222>   (30)..(30)
<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (32)..(32)
<223>   A stretch of 3 proteinogenic amino-acids, non-proteinogenic amino
        acids, D-amino acids, peptidomimetic building blocks or combinations
        thereof

<220>
<221>   MISC_FEATURE
<222>   (34)..(34)
<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (38)..(38)
<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (41)..(41)
<223>   One proteinogenic amino-acid, non-proteinogenic amino acid, D-amino
        acid or peptidomimetic building block

<220>
<221>   MISC_FEATURE
<222>   (43)..(43)
<223>   One proteinogenic amino-acid, non-proteinogenic amino acid, D-amino
        acid or peptidomimetic building block

<220>
<221>   MISC_FEATURE
<222>   (45)..(45)
<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (48)..(48)
```

<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (53)..(53)
<223>   One proteinogenic amino-acid, non-proteinogenic amino acid, D-amino
        acid or peptidomimetic building block

<220>
<221>   MISC_FEATURE
<222>   (55)..(55)
<223>   A stretch of 2 proteinogenic amino-acids, non-proteinogenic amino
        acids, D-amino acids, peptidomimetic building blocks or combinations
        thereof

<220>
<221>   MISC_FEATURE
<222>   (60)..(60)
<223>   A stretch of 3 proteinogenic amino-acids, non-proteinogenic amino
        acids, D-amino acids, peptidomimetic building blocks or combinations
        thereof

<220>
<221>   MISC_FEATURE
<222>   (62)..(62)
<223>   A stretch of 5 proteinogenic amino-acids, non-proteinogenic amino
        acids, D-amino acids, peptidomimetic building blocks or combinations
        thereof

<220>
<221>   MISC_FEATURE
<222>   (55)..(55)
<223>   May or may not be present
        A stretch of proteinogenic amino-acids, non-proteinogenic amino acids,
        D-amino acids, peptidomimetic building blocks or combinations thereof

<400>   4
Xaa Thr Leu Xaa Arg Leu Arg Asn Tyr Leu Lys Lys Gly Thr Lys Xaa
1               5                   10                  15

Ala Xaa Phe Glu Lys Xaa Val Ile Leu Xaa Tyr Xaa Thr Xaa Leu Xaa
        20                  25                  30

Leu Xaa Asp Arg Lys Xaa Glu Leu Xaa Gly Xaa Met Xaa Thr Tyr Xaa
        35                  40                  45

Lys Lys Gly Glu Xaa Lys Xaa Tyr Val Ile Asn Xaa Lys Xaa Tyr Xaa
    50                  55                  60

<210>   5
<211>   8
<212>   PRT
<213>   Artificial Sequence

<220>
<223>   Flag epitope tag

<400>   5

```
Asp Tyr Lys Asp Asp Asp Asp Lys
1               5
```

```
<210>   6
<211>   23
<212>   DNA
<213>   Artificial Sequence

<220>
<223>   CHIPS 1-121 oligonucleotide primer 1

<400>   6
tttacttttg aaccgtttcc tac                              23

<210>   7
<211>   32
<212>   DNA
<213>   Artificial Sequence

<220>
<223>   CHIPS 1-121 oligonucleotide primer 2

<400>   7
cgtcctgaat tcttagtatg catattcatt ag                    32

<210>   8
<211>   20
<212>   DNA
<213>   Artificial Sequence

<220>
<223>   CHIPS 31-121 oligonucleotide primer 1

<400>   8
aatagtggtc ttcctacaac                                  20

<210>   9
<211>   32
<212>   DNA
<213>   CHIPS 31-121 oligonucleotide primer 2

<400>   9
cgtcctgaat tcttagtatg catattcatt ag                    32

<210>   10
<211>   29
<212>   DNA
<213>   Artificial Sequence

<220>
<223>   CHIPS 31-121 pET-22b oligonucleotide primer 1

<400>   10
cgatggccaa tagtggtctt cctacaacg                        29

<210>   11
<211>   35
<212>   DNA
<213>   Artificial Sequence

<220>
```

<223>    CHIPS 31-121 pET-22b oligonucleotide primer 2

<400>    11
ctactagctg aattcttagt atgcatattc attag                                    35

<210>    12
<211>    37
<212>    PRT
<213>    C5a amino acids 1-37

<400>    12

```
Met Asn Ser Phe Asn Tyr Thr Thr Pro Asp Tyr Gly His Tyr Asp Asp
1               5                   10                  15

Lys Asp Thr Leu Asp Leu Asn Thr Pro Val Asp Lys Thr Ser Asn Thr
        20                  25                  30

Leu Arg Val Pro Asp
        35
```

<210>    13
<211>    313
<212>    PRT
<213>    C5a amino acids 38-350

<400>    13

```
Ile Leu Ala Leu Val Ile Phe Ala Val Val Phe Leu Val Gly Val Leu
1               5                   10                  15

Gly Asn Ala Leu Val Val Trp Val Thr Ala Phe Glu Ala Lys Arg Thr
        20                  25                  30

Ile Asn Ala Ile Trp Phe Leu Asn Leu Ala Val Ala Asp Phe Leu Ser
        35                  40                  45

Cys Leu Ala Leu Pro Ile Leu Phe Thr Ser Ile Val Gln His His His
        50                  55                  60

Trp Pro Phe Gly Gly Ala Ala Cys Ser Ile Leu Pro Ser Leu Ile Leu
65                  70                  75                  80

Leu Asn Met Tyr Ala Ser Ile Leu Leu Ala Thr Ile Ser Ala Asp
                85                  90                  95

Arg Phe Leu Leu Val Phe Lys Pro Ile Trp Cys Gln Asn Phe Arg Gly
            100                 105                 110

Ala Gly Leu Ala Trp Ile Ala Cys Ala Val Ala Trp Gly Leu Ala Leu
            115                 120                 125

Leu Leu Thr Ile Pro Ser Phe Leu Tyr Arg Val Val Arg Glu Glu Tyr
            130                 135                 140

Phe Pro Pro Lys Val Leu Cys Gly Val Asp Tyr Ser His Asp Lys Arg
145                 150                 155                 160

Arg Glu Arg Ala Val Ala Ile Val Arg Leu Val Leu Gly Phe Leu Trp
                165                 170                 175

Pro Leu Leu Thr Leu Thr Ile Cys Tyr Thr Phe Ile Leu Leu Arg Thr
            180                 185                 190
```

```
Trp Ser Arg Arg Ala Thr Arg Ser Thr Lys Thr Leu Lys Val Val Val
        195             200             205

Ala Val Val Ala Ser Phe Phe Ile Phe Trp Leu Pro Tyr Gln Val Thr
        210             215             220

Gly Ile Met Met Ser Phe Leu Glu Pro Ser Ser Pro Thr Phe Leu Leu
225             230             235             240

Leu Asn Lys Leu Asp Ser Leu Cys Val Ser Phe Ala Thr Ile Asn Cys
            245             250             255

Cys Ile Asn Pro Ile Ile Tyr Val Val Ala Gly Gln Gly Phe Gln Gly
            260             265             270

Arg Leu Arg Lys Ser Leu Pro Ser Leu Leu Arg Asn Val Leu Thr Glu
        275             280             285

Glu Ser Val Val Arg Glu Ser Lys Ser Phe Thr Arg Ser Thr Val Asp
        290             295             300

Thr Met Ala Gln Lys Thr Gln Ala Val
305             310
```

<210> 14
<211> 91
<212> PRT
<213> Artificial Sequence

<220>
<223> CHIPS amino acids 31-121

<400> 14

```
Asn Ser Gly Leu Pro Thr Thr Leu Gly Lys Leu Asp Glu Arg Leu Arg
1           5               10              15

Asn Tyr Leu Lys Lys Gly Thr Lys Asn Ser Ala Gln Phe Glu Lys Met
            20              25              30

Val Ile Leu Thr Glu Asn Lys Gly Tyr Tyr Thr Val Tyr Leu Asn Thr
            35              40              45

Pro Leu Ala Glu Asp Arg Lys Asn Val Glu Leu Leu Gly Lys Met Tyr
        50              55              60

Lys Thr Tyr Phe Phe Lys Lys Gly Glu Ser Lys Ser Ser Tyr Val Ile
65              70              75              80

Asn Gly Pro Gly Lys Thr Asn Glu Tyr Ala Tyr
                85              90
```